# EUROPEAN PATENT SPECIFICATION

(11) **EP 4 203 877 B1**
(45) Date of publication and mention of the grant of the patent: **05.11.2025**
(21) Application number: 21777394.4
(22) Date of filing: 12.08.2021
(51) Int. Cl.: A61F 9/008

(54) **SYSTEM FOR CLEARING AN OBSTRUCTION FROM THE PATH OF A SURGICAL LASER**
SYSTEM ZUM ENTFERNEN EINES HINDERNISSES AUS DEM WEG EINES CHIRURGISCHEN LASERS
SYSTÈME PERMETTANT D'ÉLIMINER UNE OBSTRUCTION DU TRAJET D'UN LASER CHIRURGICAL

(30) Priority: 25.08.2020 US 202063070228 P; 15.03.2021 US 202117202257
(43) Date of publication of application: 05.07.2023
(73) Proprietor: Vialase, Inc., Aliso Viejo, CA 92656 (US)
(72) Inventor: HOLLAND, Guy, San Juan Capistrano, CA 92675 (US); JUHASZ, Tibor, San Clemente, CA 92672 (US); MIKULA, Eric R., Aliso Viejo, CA 92656 (US); RAKSI, Ferenc, Mission Viejo, CA 92691 (US); SHARMA, Manu, Ladera Ranch, CA 92694 (US); SRASS, Hadi, Yorba Linda, CA 92886 (US); SUAREZ, Carlos, Tustin, CA 92782 (US)
(74) Representative: Hoefer & Partner Patentanwälte mbB
(86) International application number: PCT/US2021/045824
(87) International publication number: WO 2022/046430

(56) References cited:
- US-A1- 2020 078 217
- US-A1- 2020 078 218

## Description

### TECHNICAL FIELD

The present disclosure relates generally to the field of medical devices and treatment of diseases in ophthalmology including glaucoma, and more particularly to systems and apparatuses for treating the trabecular meshwork and Schlemm's canal using a laser.

### BACKGROUND

Before describing the different types of glaucoma and current diagnosis and treatments options, a brief overview of the anatomy of the eye is provided.

### Anatomy of the Eye

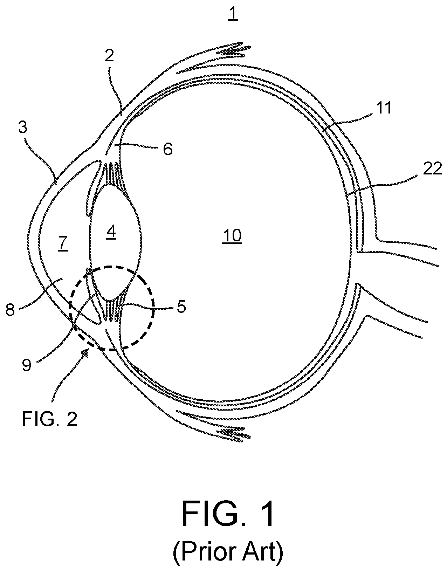

With reference to FIGS. 1-3, the outer tissue layer of the eye **1** includes a sclera **2** that provides the structure of the eye's shape. In front of the sclera **2** is a cornea **3** that is comprised of transparent layers of tissue that allow light to enter the interior of the eye. Inside the eye **1** is a crystalline lens **4** that is connected to the eye by fiber zonules **5,** which are connected to the ciliary body **6.** Between the crystalline lens **4** and the cornea **3** is an anterior chamber **7** that contains a flowing clear liquid called aqueous humor **8.** Encircling the perimeter of the crystalline lens **4** is an iris **9** which forms a pupil around the approximate center of the crystalline lens. A posterior chamber **23** is an annular volume behind the iris **9** and bounded by the ciliary body **6,** fiber zonules **5,** and the crystalline lens **4.** The vitreous humor **10** is located between the crystalline lens **4** and the retina **11.** Light entering the eye is optically focused through the cornea **3** and crystalline lens.

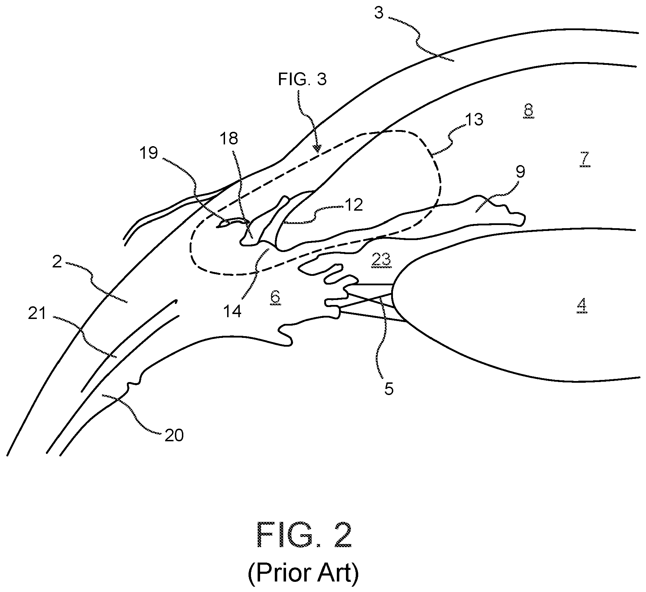

With reference to FIG. 2, the corneoscleral junction of the eye is the portion of the anterior chamber **7** at the intersection of the iris **9,** the sclera **2,** and the cornea **3.** The anatomy of the eye **1** at the corneoscleral junction includes a trabecular meshwork **12.** The trabecular meshwork **12** is a fibrous network of tissue that encircles the iris **9** within the eye **1.** In simplified, general terms the tissues of the corneoscleral junction are arranged as follows: the iris **9** meets the ciliary body **6,** the ciliary body meets with the underside of the scleral spur **14,** the top of the scleral spur serves as an attachment point for the bottom of the trabecular meshwork **12.** The ciliary body is present mainly in the posterior chamber, but also extends into the very corner of the anterior chamber **7.** The network of tissue layers that make up the trabecular meshwork **12** are porous and thus present a pathway for the egress of aqueous humor **8** flowing from the anterior chamber **7.** This pathway may be referred to herein as an aqueous humor outflow pathway, an aqueous outflow pathway, or simply an outflow pathway.

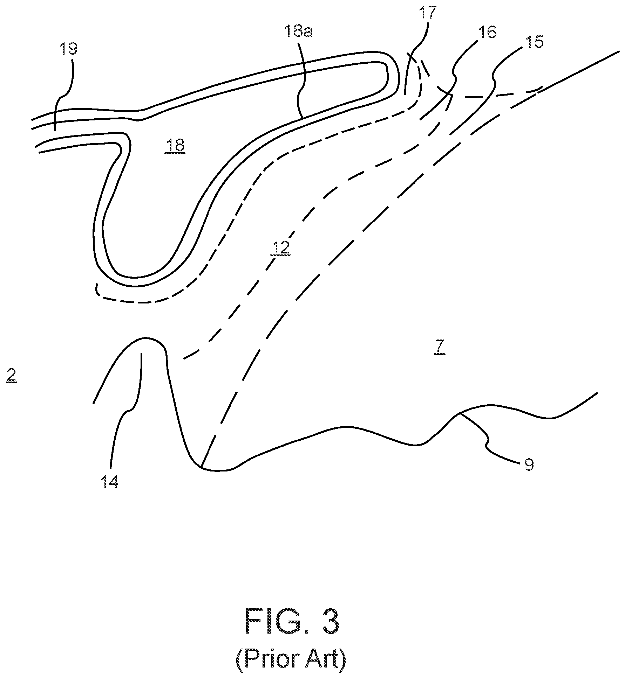

Referring to FIG. 3, the pathway formed by the pores in the trabecular meshwork **12** connect to a set of thin, porous tissue layers called the uveal **15,** the corneoscleral meshwork **16,** and the juxtacanalicular tissue **17.** The juxtacanalicular tissue **17,** in turn, abuts a structure called Schlemm's canal **18.** The Schlemm's canal **18** carries a mixture of aqueous humor **8** and blood from the surrounding tissue to drain into the venous system though a system of collector channels **19.** As shown in FIG. 2, the vascular layer of the eye, referred to as the choroid **20,** is next to the sclera **2.** A space, called the suprachoroidal space **21,** may be present between the choroid **20** and the sclera **2.** The general region near the periphery of the wedge between the cornea **3** and the iris **9,** running circumferentially is called the irido-corneal angle **13.** The irido-corneal angle **13** may also be referred to as the corneal angle of the eye or simply the angle of the eye. The ocular tissues illustrated in FIG. 3 are all considered to be within the irido-corneal angle **13.**

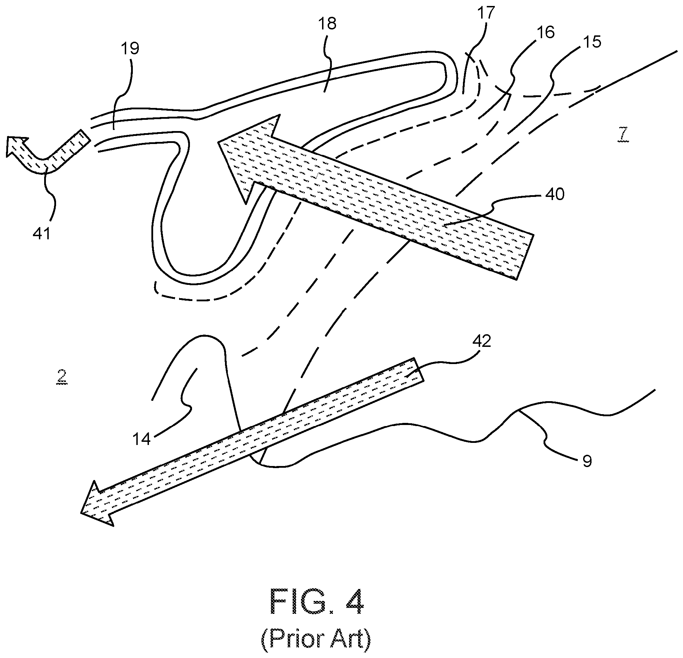

With reference to FIG. 4, two possible outflow pathways for the movement of aqueous humor **8** include a trabecular outflow pathway **40** and a uveoscleral outflow pathway **42.** Aqueous humor **8,** which is produced by the ciliary body **6,** flows from the posterior chamber **23** through the pupil into the anterior chamber **7,** and then exits the eye through one or more of the two different outflow pathways **40, 42.** Approximately 90% of the aqueous humor **8** leaves via the trabecular outflow pathway **40** by passing through the trabecular meshwork **12,** into the Schlemm's canal **18** and through one or more plexus of collector channels **19** before draining through a drain path **41** into the venous system. Any remaining aqueous humor **8** leaves primarily through the uveoscleral outflow pathway **42.** The uveoscleral outflow pathway **42** passes through the ciliary body **6** face and iris root into the suprachoroidal space **21** (shown in FIG. 2). Aqueous humor 8 drains from the suprachoroidal space **21,** from which it can be drained through the sclera **2.**

The intra-ocular pressure of the eye depends on the aqueous humor **8** outflow through the trabecular outflow pathway **40** and the resistance to outflow of aqueous humor through the trabecular outflow pathway. The intra-ocular pressure of the eye is largely independent of the aqueous humor **8** outflow through the uveoscleral outflow pathway **42.** Resistance to the outflow of aqueous humor **8** through the trabecular outflow pathway **40** may lead to elevated intra-ocular pressure of the eye, which is a widely recognized risk factor for glaucoma. Resistance through the trabecular outflow pathway **40** may increase due a collapsed or malfunctioning Schlemm's canal **18** and trabecular meshwork **12.**

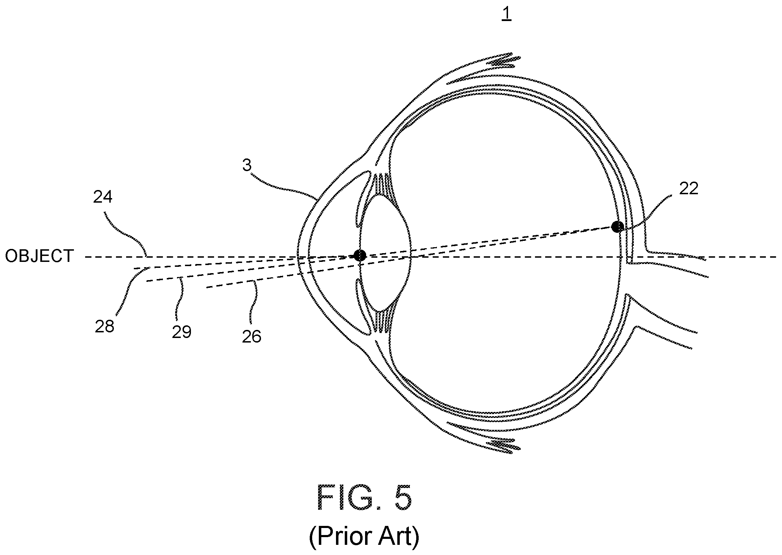

Referring to FIG. 5, as an optical system, the eye **1** is represented by an optical model described by idealized centered and rotationally symmetrical surfaces, entrance and exit pupils, and six cardinal points: object and image space focal points, first and second principal planes, and first and second nodal points. Angular directions relative to the human eye are often defined with respect to an optical axis **24,** a visual axis **26,** a pupillary axis **28** and a line of sight **29** of the eye. The optical axis **24** is the symmetry axis, the line connecting the vertices of the idealized surfaces of the eye. The visual axis **26** connects the foveal center **22** with the first and second nodal points to the object. The line of sight **29** connects the fovea through the exit and entrance pupils to the object. The pupillary axis **28** is normal to the anterior surface of the cornea **3** and directed to the center of the entrance pupil. These axes of the eye differ from one another only by a few degrees and fall within a range of what is generally referred to as the direction of view.

### Glaucoma

Glaucoma is a group of diseases that can harm the optic nerve and cause vision loss or blindness. It is the leading cause of irreversible blindness. Approximately 80 million people are estimated to have glaucoma worldwide and of these, approximately 6.7 million are bilaterally blind. More than 2.7 million Americans over age 40 have glaucoma. Symptoms start with loss of peripheral vision and can progress to blindness.

There are two forms of glaucoma, one is referred to as closed-angle glaucoma, the other as open-angled glaucoma. With reference to FIGS. 1-4, in closed-angle glaucoma, the iris **9** in a collapsed anterior chamber **7** may obstruct and close off the flow of aqueous humor **8.** In open-angle glaucoma, which is the more common form of glaucoma, the permeability of ocular tissue may be affected by irregularities in the juxtacanalicular tissue **17** and inner wall of Schlemm's canal **18a,** blockage of tissue in the irido-corneal angle **13** along the trabecular outflow pathway **40.**

As previously stated, elevated intra-ocular pressure (IOP) of the eye, which damages the optic nerve, is a widely recognized risk factor for glaucoma. However, not every person with increased eye pressure will develop glaucoma, and glaucoma can develop without increased eye pressure. Nonetheless, it is desirable to reduce elevated IOP of the eye to reduce the risk of glaucoma.

Methods of diagnosing conditions of the eye of a patient with glaucoma include visual acuity tests and visual field tests, dilated eye exams, tonometry, i.e. measuring the intra-ocular pressure of the eye, and pachymetry, i.e. measuring the thickness of the cornea. Deterioration of vision starts with the narrowing of the visual field and progresses to total blindness. Imaging methods include slit lamp examination, observation of the irido-corneal angle with a gonioscopic lens and optical coherence tomography (OCT) imaging of the anterior chamber and the retina.

Once diagnosed, some clinically proven treatments are available to control or lower the intra-ocular pressure of the eye to slow or stop the progress of glaucoma. The most common treatments include: 1) medications, such as eye drops or pills, 2) laser surgery, and 3) traditional surgery. Treatment usually begins with medication. However, the efficacy of medication is often hindered by patient non-compliance. When medication does not work for a patient, laser surgery is typically the next treatment to be tried. Traditional surgery is invasive, more high risk than medication and laser surgery, and has a limited time window of effectiveness. Traditional surgery is thus usually reserved as a last option for patients whose eye pressure cannot be controlled with medication or laser surgery.

### Laser Surgery

With reference to FIG. 2, laser surgery for glaucoma targets the trabecular meshwork **12** to decrease aqueous humor **8** flow resistance. Common laser treatments include Argon Laser Trabeculoplasty (ALT), Selective Laser Trabeculoplasty (SLT) and Excimer Laser Trabeculostomy (ELT).

ALT was the first laser trabeculoplasty procedure. During the procedure, an argon laser of 514 nm wavelength is applied to the trabecular meshwork **12** around 180 degrees of the circumference of the irido-corneal angle **13.** The argon laser induces a thermal interaction with the ocular tissue that produces openings in the trabecular meshwork **12.** ALT, however, causes scarring of the ocular tissue, followed by inflammatory responses and tissue healing that may ultimately close the opening through the trabecular meshwork **12** formed by the ALT treatment, thus reducing the efficacy of the treatment. Furthermore, because of this scarring, ALT therapy is typically not repeatable.

SLT is designed to lower the scarring effect by selectively targeting pigments in the trabecular meshwork **12** and reducing the amount of heat delivered to surrounding ocular tissue. During the procedure, a solid-state laser of 532 nm wavelength is applied to the trabecular meshwork **12** between 180 to 360 degrees around the circumference of the irido-corneal angle **13** to remove the pigmented cells lining the trabeculae which comprise the trabecular meshwork. The collagen ultrastructure of the trabecular meshwork is preserved during SLT. **12.** SLT treatment can be repeated, but subsequent treatments have lower effects on IOP reduction.

ELT uses a 308 nm wavelength ultraviolet (UV) excimer laser and non-thermal interaction with ocular tissue to treat the trabecular meshwork **12** and inner wall of Schlemm's canal in a manner that does not invoke a healing response. Therefore, the IOP lowering effect lasts longer. However, because the UV light of the laser cannot penetrate deep into the eye, the laser light is delivered to the trabecular meshwork **12** via an optical fiber inserted into the eye **1** through an opening and the fiber is brought into contact with the trabecular meshwork. The procedure is highly invasive and is generally practiced simultaneously with cataract procedures when the eye is already surgically open. Like ALT and SLT, ELT also lacks control over the amount of IOP reduction.

None of these existing laser treatments represents an ideal treatment for glaucoma. Accordingly, what is needed are systems, apparatuses, and methods for laser surgery treatment of glaucoma that non-invasively provide long term reduction of IOP without significant scarring of tissue, so the treatment may be completed in a single procedure and repeated at a later time if necessary.

The use of femtosecond lasers for surgery of the trabecular meshwork in the treatment of glaucoma is disclosed in various related patent applications identified above, including U.S. Patent Application Serial No. 16/674,850, filed November 5, 2019,
published No. US 2020/0078218 A1, for "Surgical System and Procedure for Treatment of the Trabecular Meshwork and Schlemm's Canal Using a Femtosecond Laser," U.S. Patent Application Serial No. 16/036,833, filed July 16, 2018, for "Integrated Surgical System and Method for Treatment in the Irido-Corneal Angle of the Eye," and U.S. Patent Application Serial No. 16/125,588, filed September 7, 2018, for "Non-Invasive and Minimally Invasive Laser Surgery for the Reduction of Intraocular Pressure in the Eye."

Femtosecond laser pulses treat tissue by a process called photodisruption in which tissue at the focus of a beam is disrupted and decomposed. The intent of treating the tissue in this manner is to create an aperture through which the intraocular pressure can be reduced. As a result of the photodisruption of a volume of tissue there may be an amount of gas and debris trapped in the trabecular meshwork. This gas and debris can obstruct the path of subsequent laser pulses that prevents the creation of a clear aperture. In the absence of a clear aperture, the reduction of the intraocular pressure in the eye is confounded.

### SUMMARY

The scope of the invention is exclusively defined by the claims which follow. In particular, the methods described are given for illustrative purpose only and are not directly part of the claimed subject-matter.

The present disclosure relates to a system for treating a target volume of ocular tissue of an irido-corneal angle of an eye. The system includes a first optical subsystem, a second optical subsystem, and a control system. The first optical subsystem includes a focusing objective configured to be coupled to the eye. The second optical subsystem includes a laser source configured to output a laser beam, and a plurality of components configured to one or more of focus, scan, and direct the laser through the focusing objective, toward the target volume of ocular tissue. The control system is coupled to the second optical subsystem and is configured to control the second optical subsystem to move a focus of a laser through the target volume of ocular tissue, and photodisrupt the target volume of ocular tissue at a plurality of spots as the focus is moved through the target volume of ocular tissue. The control system controls the second optical subsystem to move the focus through the target volume of ocular tissue by causing the second optical subsystem to transverse scan the focus between at least one of: a first circumferential boundary and a second circumferential boundary of the target volume of ocular tissue, and a first azimuthal boundary and a second azimuthal boundary of the target volume of ocular tissue, and axial scan the focus between a distal extent and a proximal extent of the target volume of ocular tissue.

It is understood that other aspects of apparatuses will become apparent to those skilled in the art from the following detailed description, wherein various aspects of apparatuses are shown and described by way of illustration. As will be realized, these aspects may be implemented in other and different forms and its several details are capable of modification in various other respects. Accordingly, the drawings and detailed description are to be regarded as illustrative in nature and not as restrictive.

### BRIEF DESCRIPTION OF THE DRAWINGS

Various aspects of systems, apparatuses, and methods will now be presented in the detailed description by way of example, and not by way of limitation, with reference to the accompanying drawings, wherein:
FIG. 1 is a sectional schematic illustration of a human eye and its interior anatomical structures.
FIG. 2 is a sectional schematic illustration of the irido-corneal angle of the eye of FIG. 1.
FIG. 3 is a sectional schematic illustration detailing anatomical structures in the irido-corneal angle of FIG. 2, including the trabecular meshwork, Schlemm's canal, and one or more collector channels branching from the Schlemm's canal.
FIG. 4 is a sectional schematic illustration of various outflow pathways for aqueous humor through the trabecular meshwork, Schlemm's canal, and collector channels of FIG. 3.
FIG. 5 is a sectional schematic illustration of a human eye showing various axes associated with the eye.
FIG. 6 is a sectional schematic illustration of an angled beam path along which one or more light beams may access the irido-corneal angle of the eye.
FIG. 7 is a block diagram of an integrated surgical system for non-invasive glaucoma surgery including a control system, a femtosecond laser source, an OCT imaging apparatus, a microscope, beam conditioners and scanners, beam combiners, a focusing objective, and a patient interface.
FIG. 8 is a detailed block diagram of the integrated surgical system of FIG. 7.
FIG. 9a and 9b are schematic illustrations of the focusing objective of the integrated surgical system of FIG. 7 coupled to (FIG. 9a) and decoupled from (FIG. 9b) the patient interface of the integrated surgical system of FIG. 7.
FIG. 9c is a schematic illustration of components of the focusing objective and the patient interface included in FIGS. 9a and 9b.
FIGS. 10a and 10b are schematic illustrations of components of the integrated surgical system of FIGS. 7 and 8 functionally arranged to form a first optical system and a second optical subsystem that enable access to the to the irido-corneal angle along the angled beam path of FIG. 6.
FIG. 10c is a schematic illustration of a beam passing through the first optical subsystem of FIGS. 10a and 10b and into the eye.
FIG. 11 is a three-dimensional schematic illustration of anatomical structures in the irido-corneal angle, including the trabecular meshwork, Schlemm's canal, a collector channel branching from the Schlemm's canal, and a surgical volume of ocular tissue to be treated by the integrated surgical system of FIG. 7.
FIGS. 12a and 12b are two-dimensional schematic illustrations of anatomical structures in the irido-corneal angle and a three-dimensional laser treatment pattern to be applied by the integrated surgical system of FIG. 7 to affect a surgical volume of ocular tissue between the Schlemm's canal and the anterior chamber as shown in FIG. 11.
FIG. 13 is a three-dimensional schematic illustration of FIG. 11 subsequent to treatment of the surgical volume of ocular tissue by a laser based on the laser treatment pattern of FIGS. 12a and 12b that forms an opening between the Schlemm's canal and the anterior chamber.
FIG. 14a and 14b are a series of schematic illustrations of a laser scanning process based on the treatment pattern of FIGS. 12a and 12b, where the scanning begins adjacent the anterior chamber and proceeds toward the Schlemm's canal.
FIGS. 15a-15g are a series of schematic illustrations of a laser scanning process based on the treatment pattern of FIGS. 12a and 12b, where the scanning begins adjacent the Schlemm's canal and proceeds toward the anterior chamber.
FIG. 16a and 16b are a series of schematic illustrations of an optional laser scanning process through the opening of FIG. 15g, where the scanning begins at the end of the opening adjacent the anterior chamber and proceeds toward the Schlemm's canal.
FIG. 17 is a flowchart of a method of treating a volume of ocular tissue.
FIG. 18 is a flowchart of a method of treating an eye comprising an anterior chamber, a Schlemm's canal, and a trabecular meshwork.
FIG. 19 is a schematic illustration of a three-dimensional laser treatment pattern formed by a number of stacked two-dimensional treatment planes or layers.
FIG. 20 is a schematic illustration of a two-dimensional treatment layer defined by an array of spots.
FIG. 21 is a ramping profile of a laser treatment characterized by a distal depth (also referred to as a distal extent or entering depth) of a laser focus, a proximal depth (also referred to as a proximal extent or exiting depth) of a laser focus, and an exiting velocity and an entering velocity of laser focus movement between the extents.
FIG. 22 is a flowchart of a method of treating a volume of ocular tissue.
FIG. 23 is a schematic illustration of a target volume of ocular tissue being scanned and treated by a laser.
FIG. 24 is a schematic illustration of a two-dimensional layer of a full scan pattern comprising a treatment pattern between blanked portions.

### DETAILED DESCRIPTION

Disclosed herein are systems, apparatuses, and methods for safely and effectively reducing intra-ocular pressure (IOP) in the eye to either treat or reduce the risk of glaucoma. The systems, apparatuses, and methods enable access to the irido-corneal angle of the eye and integrate laser surgery techniques with high resolution imaging to precisely diagnose, locate, and treat abnormal ocular tissue conditions within the irido-corneal angle that may be causing elevated IOP.

A surgical system disclosed herein is configured to treat a target volume of ocular tissue of an irido-corneal angle of an eye. The surgical system includes a first optical subsystem and a second optical subsystem. The first optical subsystem includes a focusing objective configured to be coupled to the eye. The second optical subsystem includes a laser source configured to output a laser beam, and a plurality of components configured to focus, scan, and direct the laser through the focusing objective, toward the target volume of ocular tissue.

The surgical system also includes a control system coupled to the second optical subsystem. The control system is configured to control the laser to: move a focus of a laser through the target volume of ocular tissue, and to photodisrupt the target volume of ocular tissue at a plurality of spots as the focus is moved through the target volume of ocular tissue. The control system moves the focus of the laser by transverse scanning the focus at a transverse-scanning velocity between at least one of: a first circumferential boundary and a second circumferential boundary of the target volume of ocular tissue, and a first azimuthal boundary and a second azimuthal boundary of the target volume of ocular tissue, and axial scanning the focus at an axial-scanning velocity between a distal extent and a proximal extent of the target volume of ocular tissue.

The laser source may be a femtosecond laser, or a picosecond laser. Such lasers provide non-thermal photo-disruption interaction with ocular tissue to avoid thermal damage to surrounding tissue. Further, unlike other surgical methods, with femtosecond laser treatment opening surface incisions penetrating the eye can be avoided, enabling a non-invasive treatment. Instead of performing the treatment in a sterile surgical room, the non-invasive treatment can be performed in a non-sterile outpatient facility.

The surgical system may also include an optical coherence tomography (OCT) imaging apparatus for imaging the target volume of ocular tissue. An additional imaging component may be included the integrated surgical system to provide direct visual observation of the irido-corneal angle along an angle of visual observation. For example, a microscope or imaging camera may be included to assist the surgeon in the process of docking the eye to the patient interface or an immobilizing device, locating ocular tissues in the eye and observing the progress of the surgery. The angle of visual observation can also be along the angled beam path to the irido-corneal angle through the cornea and the anterior chamber.

Images from the OCT imaging apparatus and the additional imaging component providing visual observation, e.g. microscope, are combined on a display device such as a computer monitor. Different images can be registered and overlaid on a single window, enhanced, processed, differentiated by false color for easier understanding. Certain features are computationally recognized by a computer processor, image recognition and segmentation algorithm can be enhanced, highlighted, marked for display. The geometry of the treatment plan can also be combined and registered with imaging information on the display device and marked up with geometrical, numerical and textual information. The same display can also be used for user input of numerical, textual and geometrical nature for selecting, highlighting and marking features, inputting location information for surgical targeting by keyboard, mouse, cursor, touchscreen, audio or other user interface devices.

### OCT Imaging

The main imaging component of the integrated surgical system disclosed herein is an OCT imaging apparatus. OCT technology may be used to diagnose, locate and guide laser surgery directed to the irido-corneal angle of the eye. For example, with reference to FIGS. 1-3, OCT imaging may be used to determine the structural and geometrical conditions of the anterior chamber **7,** to assess possible obstruction of the trabecular outflow pathway **40** and to determine the accessibility of the ocular tissue for treatment. As previously described, the iris **9** in a collapsed anterior chamber **7** may obstruct and close off the flow of aqueous humor **8,** resulting in closed-angle glaucoma. In open-angle glaucoma, where the macroscopic geometry of the angle is normal, the permeability of ocular tissue may be affected, by blockage of tissue along the trabecular outflow pathway **40** or by the collapse of the Schlemm's canal **18** or collector channels **19.**

OCT imaging can provide the necessary spatial resolution, tissue penetration and contrast to resolve microscopic details of ocular tissue. When scanned, OCT imaging can provide two-dimensional (2D) cross-sectional images of the ocular tissue. As another aspect of the integrated surgical system, 2D cross-sectional images may be processed and analyzed to determine the size, shape and location of structures in the eye for surgical targeting. It is also possible to reconstruct three-dimensional (3D) images from a multitude of 2D cross-sectional images but often it is not necessary. Acquiring, analyzing and displaying 2D images is faster and can still provide all information necessary for precise surgical targeting.

OCT is an imaging modality capable of providing high resolution images of materials and tissue. Imaging is based on reconstructing spatial information of the sample from spectral information of scattered light from within the sample. Spectral information is extracted by using an interferometric method to compare the spectrum of light entering the sample with the spectrum of light scattered from the sample. Spectral information along the direction that light is propagating within the sample is then converted to spatial information along the same axis via the Fourier transform. Information lateral to the OCT beam propagation is usually collected by scanning the beam laterally and repeated axial probing during the scan. 2D and 3D images of the samples can be acquired this way. Image acquisition is faster when the interferometer is not mechanically scanned in a time domain OCT, but interference from a broad spectrum of light is recorded simultaneously, this implementation is called a spectral domain OCT. Faster image acquisition may also be obtained by scanning the wavelength of light rapidly from a wavelength scanning laser in an arrangement called a swept-source OCT.

The axial spatial resolution limit of the OCT is inversely proportional to the bandwidth of the probing light used. Both spectral domain and swept source OCTs are capable of axial spatial resolution below 5 micrometers (µm) with sufficiently broad bandwidth of 100 nanometers (nm) or more. In the spectral domain OCT, the spectral interference pattern is recorded simultaneously on a multichannel detector, such as a charge coupled device (CCD) or complementary metal oxide semiconductor (CMOS) camera, while in the swept source OCT the interference pattern is recorded in sequential time steps with a fast optical detector and electronic digitizer. There is some acquisition speed advantage of the swept source OCT but both types of systems are evolving and improving rapidly, and resolution and speed is sufficient for purposes of the integrated surgical system disclosed herein. Stand-alone OCT systems and OEM components are now commercially available from multiple vendors, such as Optovue Inc., Fremont, CA, Topcon Medical Systems, Oakland, NJ, Carl Zeiss Meditec AG, Germany, Nidek, Aichi, Japan, Thorlabs, Newton, NJ, Santec, Aichi, Japan, Axsun, Billercia, MA, and other vendors.

### Femtosecond Laser Source

The preferred surgical component of the integrated surgical system disclosed herein is a femtosecond laser. A femtosecond laser provides highly localized, non-thermal photo-disruptive laser-tissue interaction with minimal collateral damage to surrounding ocular tissue. Photo-disruptive interaction of the laser is utilized in optically transparent tissue. The principal mechanism of laser energy deposition into the ocular tissue is not by absorption but by a highly nonlinear multiphoton process. This process is effective only at the focus of the pulsed laser where the peak intensity is high. Regions where the beam is traversed but not at the focus are not affected by the laser. Therefore, the interaction region with the ocular tissue is highly localized both transversally and axially along the laser beam. The process can also be used in weakly absorbing or weakly scattering tissue. While femtosecond lasers with photo-disruptive interactions have been successfully used in ophthalmic surgical systems and commercialized in other ophthalmic laser procedures, none have been used in an integrated surgical system that accesses the irido-corneal angle.

In known refractive procedures, femtosecond lasers are used to create corneal flaps, pockets, tunnels, arcuate incisions, lenticule shaped incisions, partial or fully penetrating corneal incisions for keratoplasty. For cataract procedures the laser creates a circular cut on the capsular bag of the eye for capsulotomy and incisions of various patterns in the lens for braking up the interior of the crystalline lens to smaller fragments to facilitate extraction. Entry incisions through the cornea opens the eye for access with manual surgical devices and for insertions of phacoemulsification devices and intra-ocular lens insertion devices. Several companies have commercialized such surgical systems, among them the IntraLase system now available from Johnson & Johnson Vision, Santa Ana, CA, The LenSx and WaveLight systems from Alcon, Fort Worth, TX, other surgical systems from Bausch and Lomb, Rochester, NY, Carl Zeiss Meditec AG, Germany, Ziemer, Port, Switzerland, and LENSAR, Orlando, FL.

These existing systems are developed for their specific applications, for surgery in the cornea, and the crystalline lens and its capsular bag and are not capable of performing surgery in the irido-corneal angle **13** for several reasons. First, the irido-corneal angle **13** is not accessible with these surgical laser systems because the irido-corneal angle is too far out in the periphery and is outside of surgical range of these systems. Second, the angle of the laser beam from these systems, which is along the optical axis **24** to the eye **1,** is not appropriate to reaching the irido-corneal angle **13,** where there is significant scattering and optical distortion at the applied wavelength. Third, any imaging capabilities these systems may have do not have the accessibility, penetration depth and resolution to image the tissue along the trabecular outflow pathway **40** with sufficient detail and contrast.

In accordance with the integrated surgical system disclosed herein, clear access to the irido-corneal angle **13** is provided along the angled beam path **30.** The tissue, e.g., cornea **3** and the aqueous humor **8** in the anterior chamber **7,** along this angled beam path **30** is transparent for wavelengths from approximately 400 nm to 2500 nm and femtosecond lasers operating in this region can be used. Such mode locked lasers work at their fundamental wavelength with Titanium, Neodymium or Ytterbium active material. Non-linear frequency conversion techniques known in the art, frequency doubling, tripling, sum and difference frequency mixing techniques, optical parametric conversion can convert the fundamental wavelength of these lasers to practically any wavelength in the above mentioned transparent wavelength range of the cornea.

Existing ophthalmic surgical systems apply lasers with pulse durations longer than 1 ns have higher photo-disruption threshold energy, require higher pulse energy and the dimension of the photo-disruptive interaction region is larger, resulting in loss of precision of the surgical treatment. When treating the irido-corneal angle **13,** however, higher surgical precision is required. To this end, the integrated surgical system may be configured to apply lasers with pulse durations from 10 femtosecond (fs) to 1 nanosecond (ns) for generating photo-disruptive interaction of the laser beam with ocular tissue in the irido-corneal angle **13.** While lasers with pulse durations shorter than 10 fs are available, such laser sources are more complex and more expensive. Lasers with the described desirable characteristics, e.g., pulse durations from 10 femtosecond (fs) to 1 nanosecond (ns), are commercially available from multiple vendors, such as Newport, Irvine, CA, Coherent, Santa Clara, CA, Amplitude Systems, Pessac, France, NKT Photonics, Birkerod, Denmark, and other vendors.

### Accessing the Irido-corneal Angle

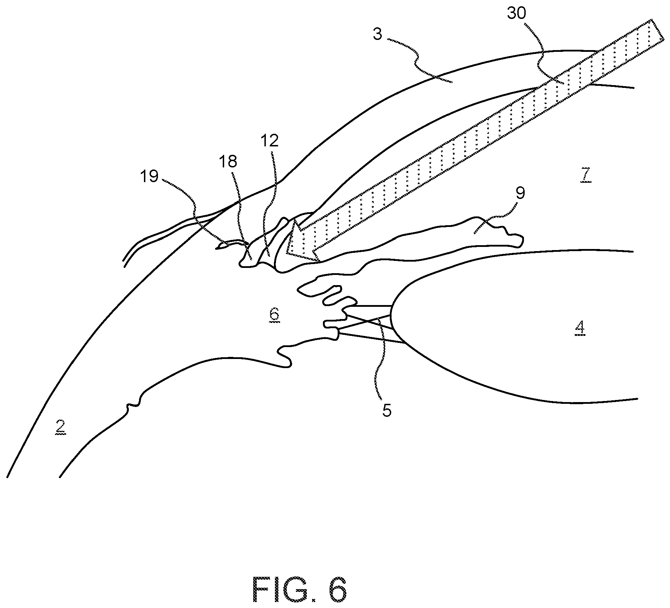

An important feature afforded by the integrated surgical system is access to the targeted ocular tissue in the irido-corneal angle **13.** With reference to FIG. 6, the irido-corneal angle **13** of the eye may be accessed via the integrated surgical system along an angled beam path **30** passing through the cornea **3** and through the aqueous humor **8** in the anterior chamber 7. For example, one or more of an imaging beam, e.g., an OCT beam and/or a visual observation beam, and a laser beam may access the irido-corneal angle **13** of the eye along the angled beam path **30.**

An optical system disclosed herein is configured to direct a light beam to an irido-corneal angle **13** of an eye along an angled beam path **30.** The optical system includes a first optical subsystem and a second optical subsystem. The first optical subsystem includes a window formed of a material with a refractive index n_{w} and has opposed concave and convex surfaces. The first optical subsystem also includes an exit lens formed of a material having a refractive index nₓ. The exit lens also has opposed concave and convex surfaces. The concave surface of the exit lens is configured to couple to the convex surface of the window to define a first optical axis extending through the window and the exit lens. The concave surface of the window is configured to detachably couple to a cornea of the eye with a refractive index n_{c} such that, when coupled to the eye, the first optical axis is generally aligned with the direction of view of the eye.

The second optical subsystem is configured to output a light beam, e.g., an OCT beam or a laser beam. The optical system is configured so that the light beam is directed to be incident at the convex surface of the exit lens along a second optical axis at an angle α that is offset from the first optical axis. The respective geometries and respective refractive indices nₓ, and n_{w} of the exit lens and window are configured to compensate for refraction and distortion of the light beam by bending the light beam so that it is directed through the cornea **3** of the eye toward the irido-corneal angle **13.** More specifically, the first optical system bends the light beam to that the light beam exits the first optical subsystem and enters the cornea **3** at an appropriate angle so that the light beam progresses through the cornea and the aqueous humor **8** in a direction along the angled beam path **30** toward the irido-corneal angle **13.**

Accessing the irido-corneal angle **13** along the angled beam path **30** provides several advantages. An advantage of this angled beam path **30** to the irido-corneal angle **13** is that the OCT beam and laser beam passes through mostly clear tissue, e.g., the cornea **3** and the aqueous humor **8** in the anterior chamber **7.** Thus, scattering of these beams by tissue is not significant. With respect to OCT imaging, this enables the use of shorter wavelength, less than approximately 1 micrometer, for the OCT to achieve higher spatial resolution. An additional advantage of the angled beam path **30** to the irido-corneal angle **13** through the cornea **3** and the anterior chamber **7** is the avoidance of direct laser beam or OCT beam light illuminating the retina **11.** As a result, higher average power laser light and OCT light can be used for imaging and surgery, resulting in faster procedures and less tissue movement during the procedure.

Another important feature provided by the integrated surgical system is access to the targeted ocular tissue in the irido-corneal angle **13** in a way that reduces beam discontinuity. To this end, the window and exit lens components of the first optical subsystem are configured to reduce the discontinuity of the optical refractive index between the cornea **3** and the neighboring material and facilitate entering light through the cornea at a steep angle.

Having thus generally described the integrated surgical system and some of its features and advantages, a more detailed description of the system and its component parts follows.

### Integrated Surgical System

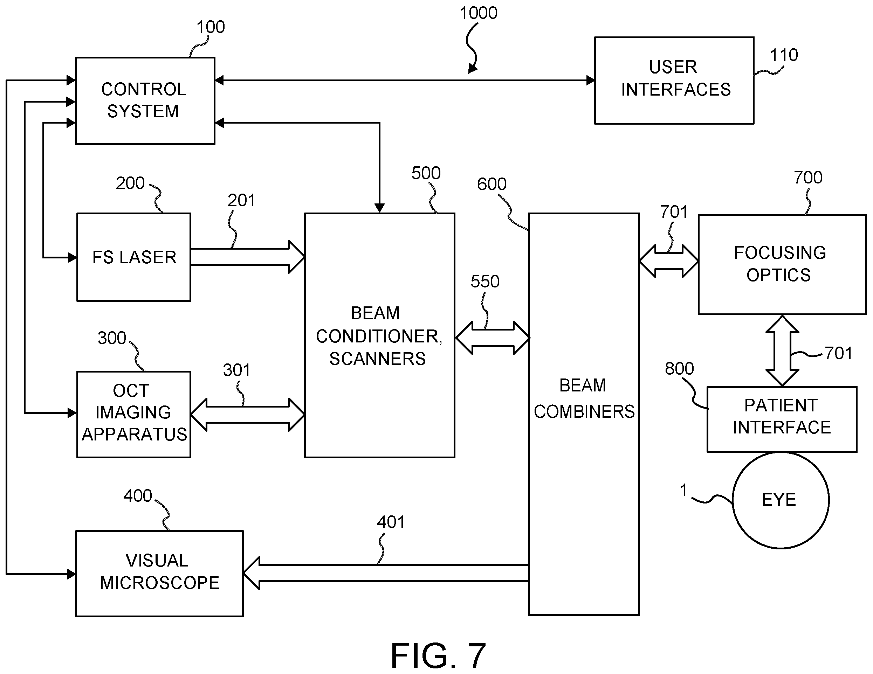

With reference to FIG. 7, an integrated surgical system **1000** for non-invasive glaucoma surgery includes a control system **100,** a surgical component **200,** a first imaging component **300** and an optional second imaging component **400.** In the embodiment of FIG. 7, the surgical component **200** is a femtosecond laser source, the first imaging component **300** is an OCT imaging apparatus, and the optional second imaging component **400** is a visual observation apparatus, e.g., a microscope, for direct viewing or viewing with a camera. Other components of the integrated surgical system **1000** include beam conditioners and scanners **500,** beam combiners **600,** a focusing objective **700,** and a patient interface **800.**

The control system **100** may be a single computer or and plurality of interconnected computers configured to control the hardware and software components of the other components of the integrated surgical system **1000.** A user interface **110** of the control system **100** accepts instructions from a user and displays information for observation by the user. Input information and commands from the user include but are not limited to system commands, motion controls for docking the patient's eye to the system, selection of pre-programmed or live generated surgical plans, navigating through menu choices, setting of surgical parameters, responses to system messages, determining and acceptance of surgical plans and commands to execute the surgical plan. Outputs from the system towards the user includes but are not limited to display of system parameters and messages, display of images of the eye, graphical, numerical and textual display of the surgical plan and the progress of the surgery.

The control system **100** is connected to the other components **200, 300, 400, 500** of the integrated surgical system **1000.** Control signals from the control system **100** to the femtosecond laser source **200** function to control internal and external operation parameters of the laser source, including for example, power, repetition rate and beam shutter. Control signals from the control system **100** to the OCT imaging apparatus **300** function to control OCT beam scanning parameters, and the acquiring, analyzing and displaying of OCT images.

Laser beams **201** from the femtosecond laser source **200** and OCT beams **301** from the OCT imaging apparatus **300** are directed towards a unit of beam conditioners and scanners **500.** Different kind of scanners can be used for the purpose of scanning the laser beam **201** and the OCT beam **301.** For scanning transversal to a beam **201, 301,** angular scanning galvanometer scanners are available for example from Cambridge Technology, Bedford, MA, Scanlab, Munich, Germany. To optimize scanning speed, the scanner mirrors are typically sized to the smallest size, which still support the required scanning angles and numerical apertures of the beams at the target locations. The ideal beam size at the scanners is typically different from the beam size of the laser beam **201** or the OCT beam **301,** and different from what is needed at the entrance of a focusing objective **700.** Therefore, beam conditioners are applied before, after or in between individual scanners. The beam conditioner and scanners **500** includes scanners for scanning the beam transversally and axially. Axial scanning changes the depth of the focus at the target region. Axial scanning can be performed by moving a lens axially in the beam path with a servo or stepper motor.

The laser beam **201** and the OCT beam **301** are combined with dichroic, polarization or other kind of beam combiners **600** to reach a common target volume or surgical volume in the eye. In an integrated surgical system **1000** having a femtosecond laser source **200,** an OCT imaging apparatus **300,** and a visual observation device **400,** the individual beams **201, 301, 401** for each of these components may be individually optimized and may be collinear or non-collinear to one another. The beam combiner **600** uses dichroic or polarization beam splitters to split and recombine light with different wavelength and/or polarization. The beam combiner **600** may also include optics to change certain parameters of the individual beams **201, 301, 401** such as beam size, beam angle and divergence. Integrated visual illumination, observation or imaging devices assist the surgeon in docking the eye to the system and identifying surgical locations.

To resolve ocular tissue structures of the eye in sufficient detail, the imaging components **300, 400** of the integrated surgical system **1000** may provide an OCT beam and a visual observation beam having a spatial resolution of several micrometers. The resolution of the OCT beam is the spatial dimension of the smallest feature that can be recognized in the OCT image. It is determined mostly by the wavelength and the spectral bandwidth of the OCT source, the quality of the optics delivering the OCT beam to the target location in the eye, the numerical aperture of the OCT beam and the spatial resolution of the OCT imaging apparatus at the target location. In one embodiment, the OCT beam of the integrated surgical system has a resolution of no more than 5 µm.

Likewise, the surgical laser beam provided by the femtosecond laser source **200** may be delivered to targeted locations with several micrometer accuracy. The resolution of the laser beam is the spatial dimension of the smallest feature at the target location that can be modified by the laser beam without significantly affecting surrounding ocular tissue. It is determined mostly by the wavelength of the laser beam, the quality of the optics delivering the laser beam to target location in the eye, the numerical aperture of the laser beam, the energy of the laser pulses in the laser beam and the spatial resolution of the laser scanning system at the target location. In addition, to minimize the threshold energy of the laser for photo-disruptive interaction, the size of the laser spot should be no more than approximately 5 µm.

It should be noted that, while the visual observation beam **401** is acquired by the visual observation device **400** using fixed, non-scanning optics, the OCT beam **301** of the OCT imaging apparatus **300** is scanned laterally in two transversal directions. The laser beam **201** of the femtosecond laser source **200** is scanned in two lateral dimensions and the depth of the focus is scanned axially.

For practical embodiments, beam conditioning, scanning and combining the optical paths are certain functions performed on the laser, OCT and visual observation optical beams. Implementation of those functions may happen in a different order than what is indicated in FIG. 7. Specific optical hardware that manipulates the beams to implement those functions can have multiple arrangements with regards to how the optical hardware is arranged. They can be arranged in a way that they manipulate individual optical beams separately, in another embodiment one component may combine functions and manipulates different beams. For example, a single set of scanners can scan both the laser beam **201** and the OCT beam **301.** In this case, separate beam conditioners set the beam parameters for the laser beam **201** and the OCT beam **301,** then a beam combiner combines the two beams for a single set of scanners to scan the beams. While many combinations of optical hardware arrangements are possible for the integrated surgical system, the following section describes in detail an example arrangement.

### Beam Delivery

In the following description, the term beam may - depending on the context - refer to one of a laser beam, an OCT beam, or a visual observation beam. A combined beam refers to two or more of a laser beam, an OCT beam, or a visual observation beam that are either collinearly combined or non-collinearly combined. Example combined beams include a combined OCT/laser beam, which is a collinear or non-colinear combination of an OCT beam and a laser beam, and a combined OCT/laser/visual beam, which is a collinear or non-collinear combination of an OCT beam, a laser beam, and a visual beam. In a collinearly combined beam, the different beams may be combined by dichroic or polarization beam splitters, and delivered along a same optical path through a multiplexed delivery of the different beams. In a non-collinear combined beam, the different beams are delivered at the same time along different optical paths that are separated spatially or by an angle between them. In the description to follow, any of the foregoing beams or combined beams may be generically referred to as a light beam. The terms distal and proximal may be used to designate the direction of travel of a beam, or the physical location of components relative to each other within the integrated surgical system. The distal direction refers to a direction toward the eye; thus an OCT beam output by the OCT imaging apparatus moves in the distal direction toward the eye. The proximal direction refers to a direction away from the eye; thus an OCT return beam from the eye moves in the proximal direction toward the OCT imaging apparatus.

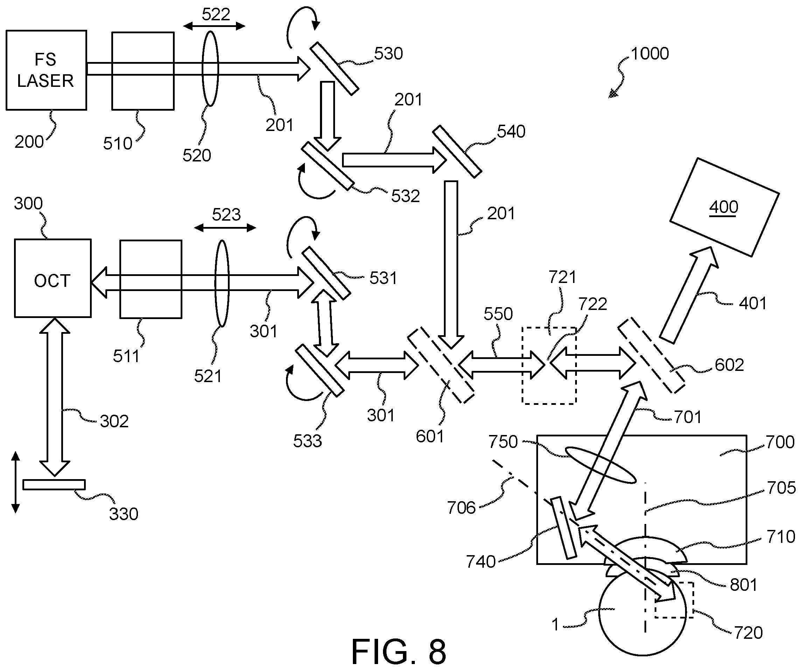

Referring to FIG. 8, an example integrated surgical system is configured to deliver each of a laser beam **201** and an OCT beam **301** in the distal direction toward an eye **1,** and receive each of an OCT return beam and the visual observation beam **401** back from the eye **1.** Regarding the delivery of a laser beam, a laser beam **201** output by the femtosecond laser source **200** passes through a beam conditioner **510** where the basic beam parameters, beam size, divergence are set. The beam conditioner **510** may also include additional functions, setting the beam power or pulse energy and shutter the beam to turn it on or off. After existing the beam conditioner **510,** the laser beam **210** enters an axial scanning lens **520.** The axial scanning lens **520,** which may include a single lens or a group of lenses, is movable in the axial direction **522** by a servo motor, stepper motor or other control mechanism. Movement of the axial scanning lens **520** in the axial direction **522** changes the axial distance of the focus of the laser beam **210** at a focal point.

In accordance with a particular embodiment of the integrated surgical system, an intermediate focal point **722** is set to fall within, and is scannable in, the conjugate surgical volume **721,** which is an image conjugate of the surgical volume **720,** determined by the focusing objective **700.** The surgical volume **720** is the spatial extent of the region of interest within the eye where imaging and surgery is performed. For glaucoma surgery, the surgical volume **720** is the vicinity of the irido-corneal angle **13** of the eye.

A pair of transverse scanning mirrors **530, 532** rotated by a galvanometer scanner scan the laser beam **201** in two essentially orthogonal transversal directions, e.g., in the x and y directions. Then the laser beam **201** is directed towards a dichroic or polarization beam splitter **540** where it is reflected toward a beam combining mirror **601** configured to combine the laser beam **201** with an OCT beam **301.**

Regarding delivery of an OCT beam, an OCT beam **301** output by the OCT imaging apparatus **300** passes through a beam conditioner **511,** an axially moveable focusing lens **521** and a transversal scanner with scanning mirrors **531** and **533.** The focusing lens **521** is used set the focal position of the OCT beam in the conjugate surgical volume **721** and the real surgical volume **720.** The focusing lens **521** is not scanned for obtaining an OCT axial scan. Axial spatial information of the OCT image is obtained by Fourier transforming the spectrum of the interferometrically recombined OCT return beam **301** and reference beams **302.** However, the focusing lens **521** can be used to readjust the focus when the surgical volume **720** is divided into several axial segments. This way the optimal imaging spatial resolution of the OCT image can be extended beyond the Rayleigh range of the OCT signal beam, at the expense of time spent on scanning at multiple ranges.

Proceeding in the distal direction toward the eye **1,** after the scanning mirrors **531** and **533,** the OCT beam **301** is combined with the laser beam **201** by the beam combiner mirror **601.** The OCT beam **301** and laser beam **201** components of the combined laser/OCT beam **550** are multiplexed and travel in the same direction to be focused at an intermediate focal point **722** within the conjugate surgical volume **721.** After having been focused in the conjugate surgical volume **721,** the combined laser/OCT beam **550** propagates to a second beam combining mirror **602** where it is combined with a visual observation beam **401** to form a combined laser/OCT/visual beam **701.**

The combined laser/OCT/visual beam **701** traveling in the distal direction then passes through a relay lens **750** included in the focusing objective **700,** is reflected by a reflecting surface **740,** which may be a planar beam-folding mirror or a facet inside an optic, and then passes through an exit lens **710** and a window **801** of a patient interface, where the intermediate focal point **722** of the laser beam within the conjugate surgical volume **721** is re-imaged into a focal point in the surgical volume **720.** The focusing objective **700** re-images the intermediate focal point **722,** through the window **801** of a patient interface, into the ocular tissue within the surgical volume **720.** In one configuration, the reflecting surface **740** in the form of a facet inside an optic may have a specialized coating for broadband reflection (visible, OCT and femtosecond) and low difference between s and p polarization group delay dispersion (GDD).

A scattered OCT return beam **301** from the ocular tissue travels in the proximal direction to return to the OCT imaging apparatus **300** along the same paths just described, in reverse order. The reference beam **302** of the OCT imaging apparatus **300,** passes through a reference delay optical path and return to the OCT imaging apparatus from a moveable mirror **330.** The reference beam **302** is combined interferometrically with the OCT return beam **301** on its return within the OCT imaging apparatus **300.** The amount of delay in the reference delay optical path is adjustable by moving the moveable mirror **330** to equalize the optical paths of the OCT return beam **301** and the reference beam **302.** For best axial OCT resolution, the OCT return beam **301** and the reference beam **302** are also dispersion compensated to equalize the group velocity dispersion within the two arms of the OCT interferometer.

When the combined laser/OCT/visual beam **701** is delivered through the cornea **3** and the anterior chamber **7,** the combined beam passes through posterior and anterior surface of the cornea at a steep angle, far from normal incidence. These surfaces in the path of the combined laser/OCT/visual beam **701** create excessive astigmatism and coma aberrations that need to be compensated for.

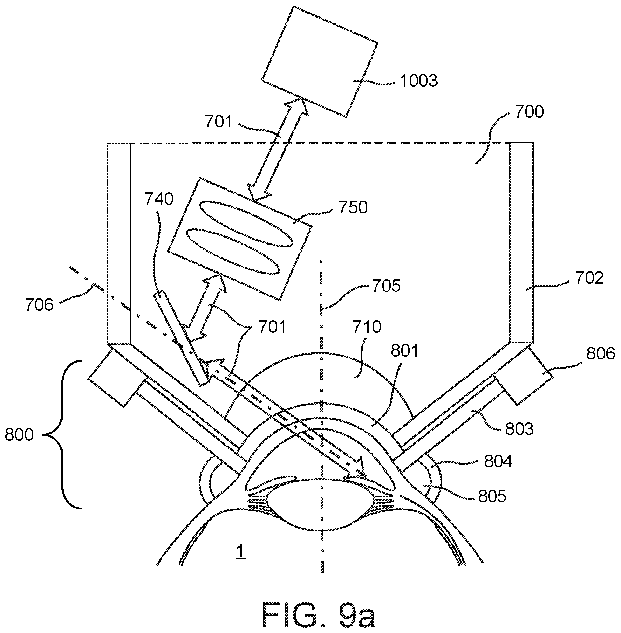

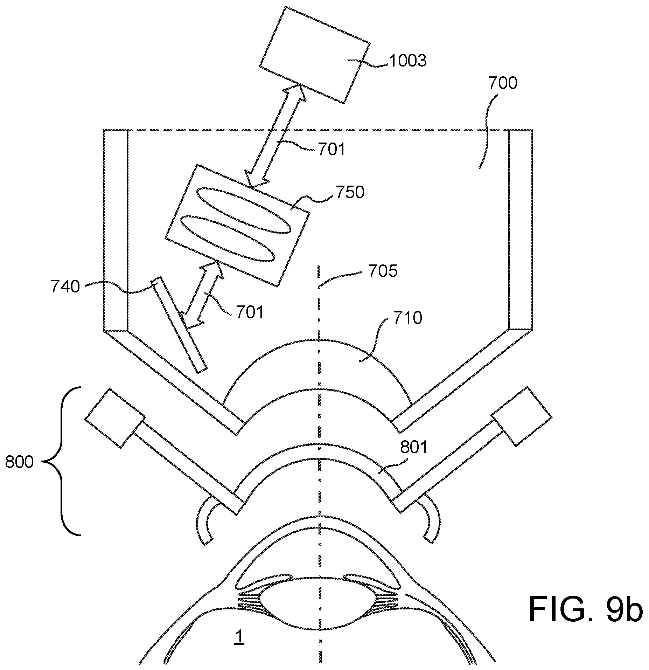

With reference to FIGS. 9a and 9b, in an embodiment of the integrated surgical system **1000,** optical components of the focusing objective **700** and patient interface **800** are configured to minimize spatial and chromatic aberrations and spatial and chromatic distortions. FIG. 9a shows a configuration when both the eye **1,** the patient interface **800** and the focusing objective **700** all coupled together. FIG. 9b shows a configuration when both the eye **1,** the patient interface **800** and the focusing objective **700** all detached from one another.

The patient interface **800** optically and physically couples the eye **1** to the focusing objective **700,** which in turn optically couples with other optic components of the integrated surgical system **1000.** The patient interface **800** serves multiple functions. It immobilizes the eye relative to components of the integrated surgical system; creates a sterile barrier between the components and the patient; and provides optical access between the eye and the instrument. The patient interface **800** is a sterile, single use disposable device and it is coupled detachably to the eye **1** and to the focusing objective **700** of the integrated surgical system **1000.**

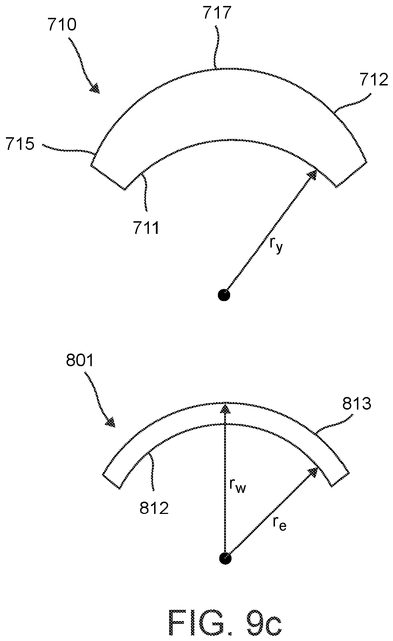

The patient interface **800** includes a window **801** having an eye-facing, concave surface **812** and an objective-facing, convex surface **813** opposite the concave surface. The window **801** thus has a meniscus form. With reference to FIG. 9c, the concave surface **812** is characterized by a radius of curvature rₑ, while the convex surface **813** is characterized by a radius of curvature r_{w}. The concave surface **812** is configured to couple to the eye, either through a direct contact or through index matching material, liquid or gel, placed in between the concave surface **812** and the eye **1.** The window **801** may be formed of glass and has a refractive index *n_{w}*. In one embodiment, the window **801** is formed of fused silica and has a refractive index *n_{w}* of 1.45. Fused silica has the lowest index from common inexpensive glasses. Fluoropolymers such as the Teflon AF are another class of low index materials that have refractive indices lower than fused silica, but their optical quality is inferior to glasses and they are relatively expensive for high volume production. In another embodiment the window **801** is formed of the common glass BK7 and has a refractive index *n_{w}* of 1.50. A radiation resistant version of this glass, BK7G18 from Schott AG, Mainz, Germany, allows gamma sterilization of the patient interface **800** without the gamma radiation altering the optical properties of the window **801.**

Returning to FIGS. 9a and 9b, the window **801** is surrounded by a wall **803** of the patient interface **800** and an immobilization device, such as a suction ring **804.** When the suction ring **804** is in contact with the eye **1,** an annular cavity **805** is formed between the suction ring and the eye. When vacuum applied to the suction ring **804** and the cavity via a vacuum tube a vacuum pump (not shown in FIG. 9a and 9b), vacuum forces between the eye and the suction ring attach the eye to the patient interface **800** during surgery. Removing the vacuum releases or detach the eye **1.**

The end of the patient interface **800** opposite the eye **1** includes an attachment interface **806** configured to attach to the housing **702** of the focusing objective **700** to thereby affix the position of the eye relative to the other components of the integrated surgical system **1000.** The attachment interface **806** can work with mechanical, vacuum, magnetic or other principles and it is also detachable from the integrated surgical system.

The focusing objective **700** includes an aspheric exit lens **710** having an eye-facing, concave surface **711** and a convex surface **712** opposite the concave surface. The exit lens **710** thus has a meniscus form. While the exit lens **710** shown in FIGS. 9a and 9b is an aspheric lens giving more design freedom, in other configurations the exit lens may be a spherical lens. Alternatively, constructing the exit lens **710** as a compound lens, as opposed to a singlet, allows more design freedom to optimize the optics while preserving the main characteristics of the optical system as presented here. With reference to FIG. 9c, the concave surface **711** is characterized by a radius of curvature r_{y}, while the convex surface **712** is characterized by an aspheric shape. The aspheric convex surface **712** in combination with the spherical concave surface **711** result in an exit lens **710** having varying thickness, with the outer perimeter edges **715** of the lens being thinner than the central, apex region **717** of the lens. The concave surface **711** is configured to couple to the convex surface **813** of the window 801. In one embodiment, the exit lens **710** is formed of fused silica and has a refractive index *nₓ* of 1.45.

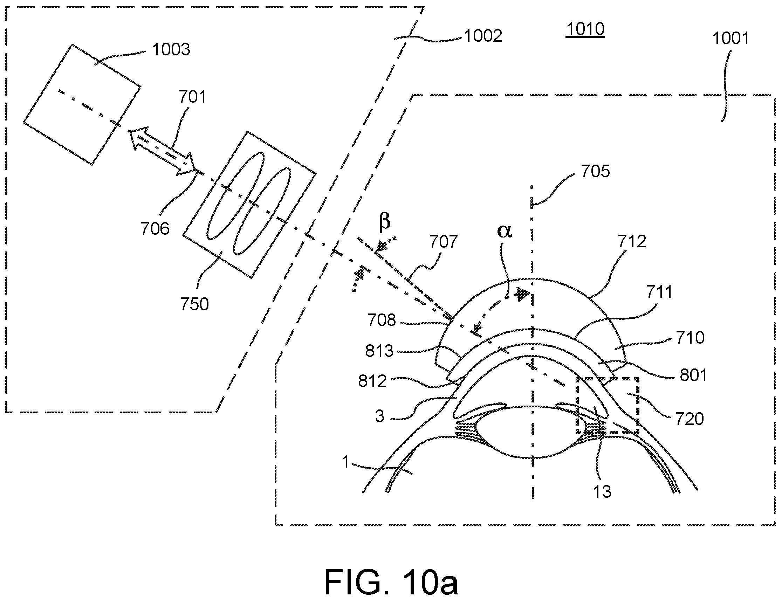

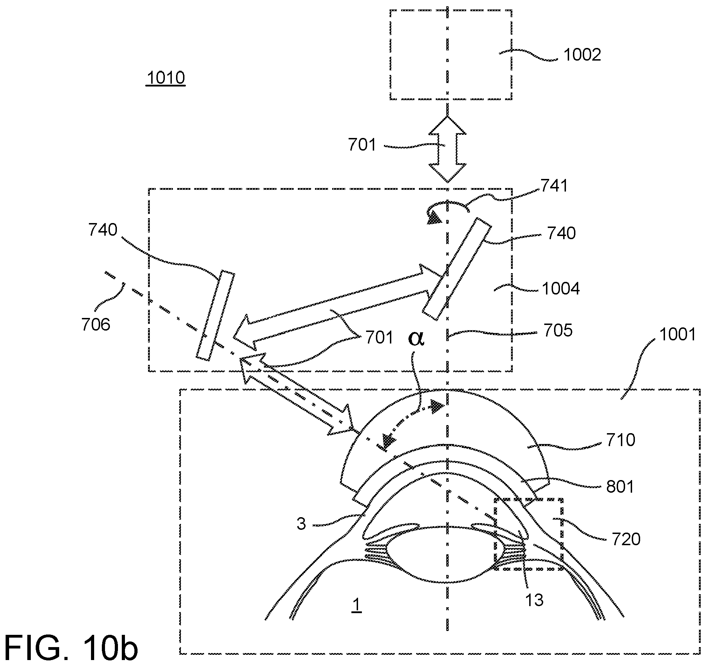

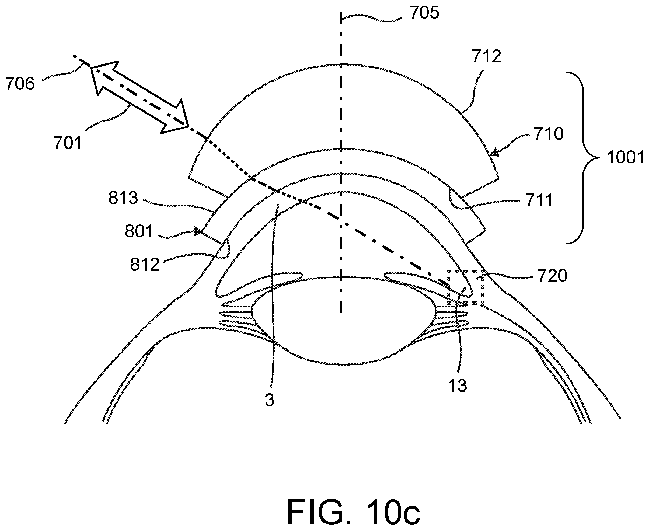

FIGS. 10a and 10b are schematic illustrations of components of the integrated surgical system of FIGS. 7 and 8 functionally arranged to form an optical system 1010 having a first optical subsystem **1001** and a second optical subsystem **1002** that enable access to a surgical volume **720** in the irido-corneal angle. Each of FIGS. 10a and 10b include components of the focusing objective **700** and the patient interface **800** of FIG. 9a. However, for simplicity, the entirety of the focusing objective and the patient interface are not included in FIGS. 10a and 10b. Also, for additional simplicity in FIG. 10a, the reflecting surface **740** of FIGS. 9a and 9b is not included and the combined laser/OCT/visual beam **701** shown in FIG. 9a is unfolded or straightened out. It is understood by those skilled in the art that adding or removing planar beam folding mirrors does not alter the principal working of the optical system formed by the first optical subsystem and the second optical subsystem. FIG. 10c is a schematic illustration of a beam passing through the first optical subsystem of FIGS. 10a and 10b.

With reference to FIG. 10a, a first optical subsystem **1001** of the integrated surgical system **1000** includes the exit lens **710** of a focusing objective **700** and the window **801** of a patient interface **800.** The exit lens **710** and the window **801** are arranged relative to each other to define a first optical axis **705.** The first optical subsystem **1001** is configured to receive a beam, e.g., a combined laser/OCT/visual beam **701,** incident at the convex surface **712** of the exit lens **710** along a second optical axis **706,** and to direct the beam toward a surgical volume **720** in the irido-corneal angle **13** of the eye.

During a surgical procedure, the first optical subsystem **1001** may be assembled by interfacing the convex surface **813** of the window **801** with the concave surface **711** of the exit lens **710.** To this end, a focusing objective **700** is docked together with a patient interface **800.** As a result, the concave surface **711** of the exit lens **710** is coupled to the convex surface **813** of the window **801.** The coupling may be by direct contact or through a layer of index matching fluid. For example, when docking the patient interface **800** to focusing objective **700,** a drop of index matching fluid can be applied between the contacting surfaces to eliminate any air gap that may be between the two surfaces **711, 813** to thereby help pass the combined laser/OCT/visual beam **701** through the gap with minimal Fresnel reflection and distortion.

In order to direct the beam toward the surgical volume **720** in the irido-corneal angle **13** of the eye, the first optical subsystem **1001** is designed to account for refraction of the beam **701** as it passes through the exit lens **710,** the window **801** and the cornea **3.** To this end, and with reference to FIG. 10c, the refractive index *nₓ* of the exit lens **710** and the refractive index *n_{w}* of the window **801** are selected in view of the refractive index *n_{c}* of the cornea **3** to cause appropriate beam bending through the first optical subsystem **1001** so that when the beam **701** exits the subsystem and passes through the cornea **3,** the beam path is generally aligned to fall within the irido-corneal angle **13.**

Continuing with reference to FIG. 10c and beginning with the interface between the window **801** and the cornea **3.** Too steep of an angle of incidence at the interface where the combined laser/OCT/visual beam **701** exits the window **801** and enters the cornea **3,** i.e., at the interface between the concave surface **812** of the window and the convex surface of the cornea **3,** can create excessive refraction and distortion. To minimize refraction and distortion at this interface, in one embodiment of the first optical subsystem **1001,** the refractive index of the window **801** is closely matched to the index of the cornea **3.** For example, as describe above with reference to FIGS. 9a and 9b, the window **801** may have a refractive index lower than 1.42 to closely match the cornea **3,** which has a refractive index of 1.36.

Excessive refraction and distortion at the interface where the combined laser/OCT/visual beam **701** exits the window **801** and enters the cornea **3** may be further compensated for by controlling the bending of the beam **701** as it passed through the exit lens **710** and the window **801.** To this end, in one embodiment of the first optical subsystem **1001** the index of refraction *n_{w}* of the window **801** is larger than each of the index of refraction *nₓ* of the exit lens **710** and the index of refraction *n_{c}* of the cornea **3.** As a result, at the interface where the combined laser/OCT/visual beam **701** exits the exit lens **710** and enters the window **801,** i.e., interface between the concave surface **711** of the exit lens and the convex surface **813** of the window, the beam passes through a refractive index change from high to low that cause the beam to bend in a first direction. Then, at the interface where the combined laser/OCT/visual beam **701** exits the window **801** and enters the cornea **3,** i.e., interface between the concave surface **812** of the exit lens and the convex surface of the cornea, the beam passes through a refractive index change from low to high that cause the beam to bend in a second direction opposite the first direction.

The shape of the window **801** is chosen to be a meniscus lens. As such, the incidence angle of light has similar values on both surfaces **812, 813** of the window **801.** The overall effect is that at the convex surface **813** the light bends away from the surface normal and at the concave surface **812** the light bends towards the surface normal. The effect is like when light passes through a plan parallel plate. Refraction on one surface of the plate is compensated by refraction on the other surface a light passing through the plate does not change its direction. Refraction at the entering, convex surface **712** of the exit lens **710** distal to the eye is minimized by setting the curvature of the entering surface such that angle of incidence *β* of light **701** at the entering surface is close to a surface normal **707** to the entering surface at the intersection point **708.**

Here, the exit lens **710,** the window **801,** and the eye **1** are arranged as an axially symmetric system with a first optical axis **705.** In practice, axial symmetry is an approximation because of manufacturing and alignment inaccuracies of the optical components, the natural deviation from symmetry of the eye and the inaccuracy of the alignment of the eye relative to the window **801** and the exit lens **710** in a clinical setting. But, for design and practical purposes the eye **1,** the window **801,** and the exit lens **710** are considered as an axially symmetric first optical subsystem **1001.**

With continued reference to FIG. 10a, a second optical subsystem **1002** is optically coupled to the first optical subsystem **1001** at an angle *α* relative to the first optical axis **705** of the first optical subsystem **1001.** The advantage of this arrangement is that both optical subsystems **1001, 1002** can be designed at a much lower numerical aperture compared to a system where all optical components are designed on axis with a common optical axis.

The second optical subsystem **1002** includes a relay lens **750** that, as previously described with reference to FIG. 8, generates a conjugate surgical volume **721** of the surgical volume **720** within the eye. The second optical subsystem **1002** includes various other components **1003.** Referring to FIG. 8, these components may include a femtosecond laser source **200,** an OCT imaging apparatus **300,** a visual observation device **400,** beam conditioners and scanners **500,** and beam combiners **600.**

The second optical subsystem **1002** may include mechanical parts (not shown) configured to rotate the entire subsystem around the first optical axis **705** of the first optical subsystem **1001.** This allows optical access to the whole 360-degree circumference of the irido-corneal angle **13** of the eye **1.**

With reference to FIG. 10b, flexibility in arranging the first and second optical subsystems **1001, 1002,** relative to each other may be provided by an optical assembly **1004** interposed between the optical output of the second optical subsystem **1002** and the optical input of the first optical subsystem **1001.** In one embodiment, the optical assembly **1004** may include one or more reflecting surfaces **740,** prisms (not shown) or optical gratings (not shown) configured to receive the optical output, e.g., combined laser/OCT/visual beam **701,** of the second optical subsystem **1002,** change or adjust the direction of the combined laser/OCT/visual beam, and direct the beam to the optical input of the first optical subsystem **1001** while preserving the angle *α* between the first optical axis **705** and the second optical axis **706.**

In another configuration, the optical assembly **1004** with the reflective surface **740** further includes mechanical parts (not shown) configured to rotate the assembly around the first optical axis **705** of the first optical subsystem **1001** while keeping the second optical subsystem **1002** stationary. Accordingly, the second optical axis **706** of the second optical subsystem **1002** can be rotated around the first optical axis **705** of the first optical subsystem **1001.** This allows optical access to the whole 360-degree circumference of the irido-corneal angle **13** of the eye **1.**

With considerations described above with reference to FIGS. 9a, 9b and 9c, the design of the first optical subsystem **1001** is optimized for angled optical access at an angle *α* relative to the first optical axis **705** of the first optical subsystem **1001.** Optical access at the angle α compensates for optical aberrations of the first optical subsystem 1001. Table 1 shows the result of the optimization at access angle *α =* 72 degrees with Zemax optical design software package. This design is a practical embodiment for image guided femtosecond glaucoma surgery.

**Table 1**

| Surface | Structure and Material | Refractive index | Radius [mm] | Center Thickness [mm] |
|---|---|---|---|---|
| concave surface **711,** convex surface **712** | Exit lens **710** of focusing objective. | 1.45 | -10 | 4.5 |
| | Fused silica | | | |
| concave surface **812,** convex surface **813** | Window **801** of patient interface. | 1.50 | -10.9 | 1.0 |
| | BK7G18 | | | |
| **3** | Cornea | 1.36 | -7.83 | 0.54 |
| **8** | Aqueous humor | 1.32 | -6.53 | 3.5 |
| Target | Ophthalmic tissue | 1.38 | N/A | 0 to 1 mm |

This design produces diffraction limited focusing of 1030 nm wavelength laser beams and 850 nm wavelength OCT beams with numerical aperture (NA) up to 0.2. In one design, the optical aberrations of the first optical subsystem are compensated to a degree that the Strehl ratio of the first optical subsystem for a beam with numerical aperture larger than 0.15 at the irido-corneal angle is larger than 0.9. In another design, the optical aberrations of the first optical subsystem are partially compensated, the remaining uncompensated aberrations of the first optical system are compensated by the second optical subsystem to a degree that the Strehl ratio of the combined first and second optical subsystem for a beam with numerical aperture larger than 0.15 at the irido-corneal angle is larger than 0.9.

### Calibration

The femtosecond laser source **200,** OCT imaging apparatus **300,** and visual observation device **400** of the integrated surgical system **1000** are first individually calibrated to ensure their internal integrity and then cross-calibrated for system integrity. The essential part of system calibration is to ensure that the when the surgical focus of a laser beam **201** is commanded to a location of a surgical volume **720,** as identified by the OCT imaging apparatus and/or the visual observation device **400,** the achieved location of the focus matches the commanded location of the focus within a certain tolerance, typically within 5 to 10 µm. Also, graphical and cursor outputs, images, overlays displayed on a user interface **110,** such as a computer monitor, and user inputs of ocular tissue surgical volume **720** locations accepted from the user interface **110** should correspond to actual locations in tissue within predetermined tolerances of similar accuracy.

One embodiment of this spatial calibration procedure starts with imaging calibrated scales and scaling magnifications of the OCT imaging apparatus **300** and/or the visual observation device **400** and their displays in a way that the scale value on the display matches the real scale of the calibration target. Then laser calibration patters are exposed or burned into transparent calibration targets, and the calibration patterns are subsequently imaged. Then, the intended patterns and the actual burned patterns are compared with the imaging system of the integrated surgical system **1000** or by a separate microscope. If they do not match within the specified tolerance, the scaling parameters of the surgical patterns are re-scaled by adjusting the scaling of the laser beam scanners. This procedure is iterated, if necessary, until all spatial calibrations are within tolerance.

### Minimally Invasive Surgical Treatments

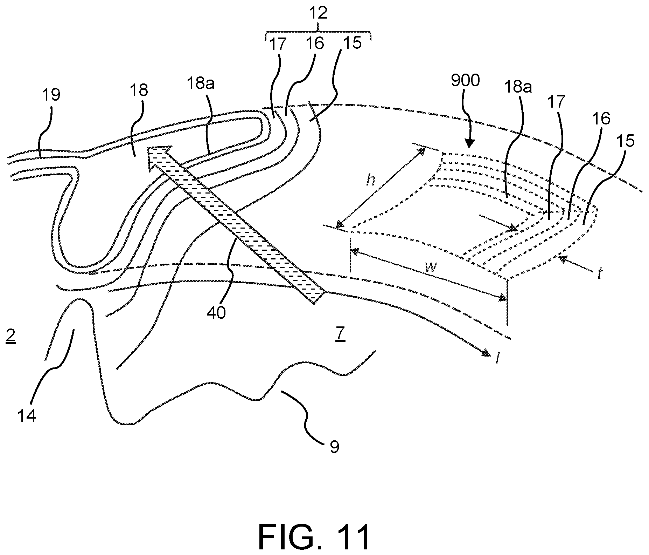

FIG. 11 is a three-dimensional schematic illustration of anatomical structures of the eye relevant to the surgical treatment enabled by the integrated surgical system **1000.** To reduce the IOP, laser treatment targets ocular tissues that affect the trabecular outflow pathway **40.** These ocular tissues may include the trabecular meshwork **12,** the scleral spur **14,** the Schlemm's canal **18,** and the collector channels **19.** The trabecular meshwork **12** has three layers, the uveal **15,** the corneoscleral meshwork **16,** and the juxtacanalicular tissue **17.** These layers are porous and permeable to aqueous, with the uveal **15** being the most porous and permeable, followed by the corneoscleral meshwork **16.** The least porous and least permeable layer of the trabecular meshwork **12** is the juxtacanalicular tissue **17.** The inner wall **18a** of the Schlemm's canal **18,** which is also porous and permeable to aqueous, has characteristics similar to the juxtacanalicular tissue **17.**

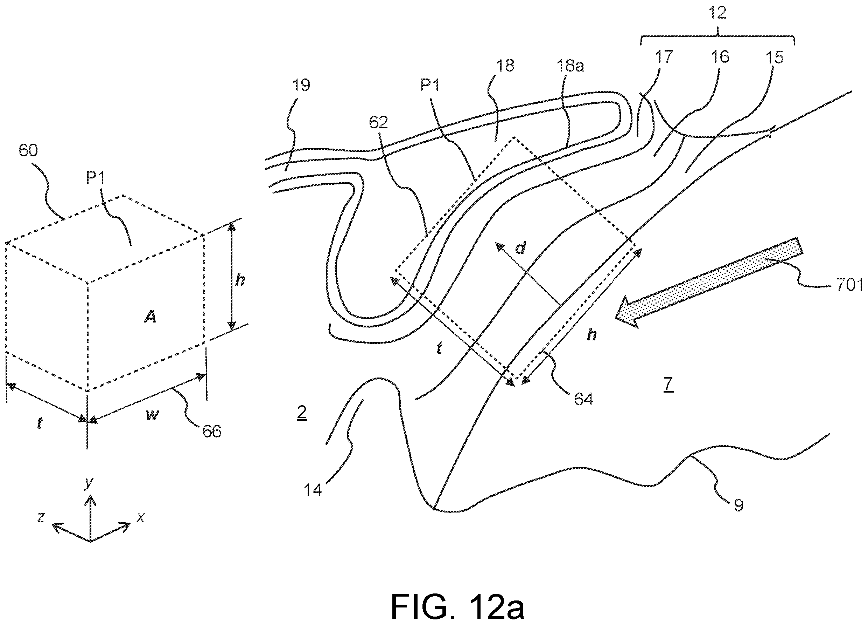

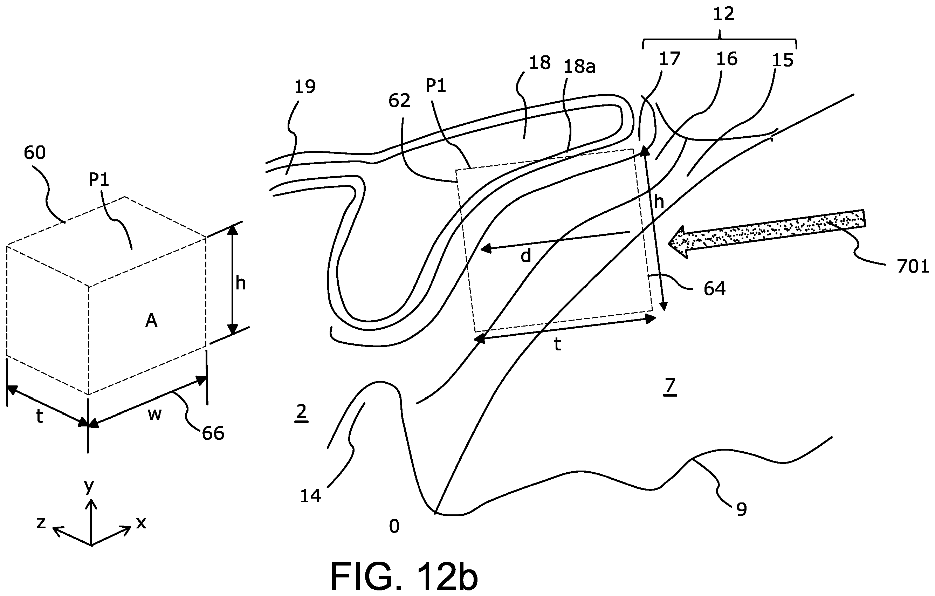

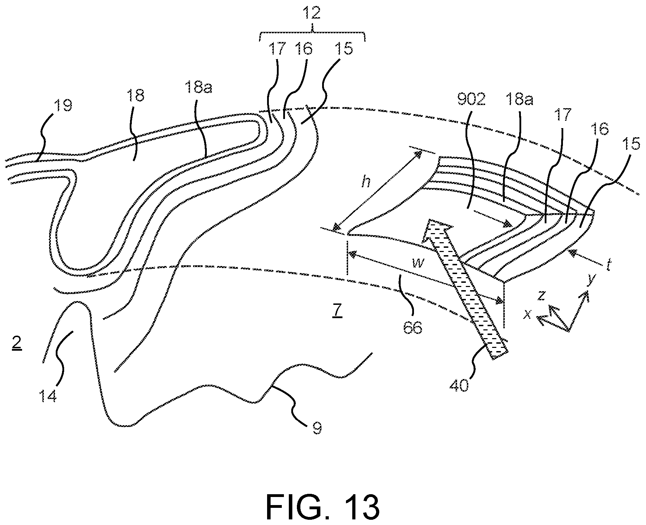

FIGS. 12a and 12b include three-dimensional illustrations of a treatment pattern **P1** to be applied by the integrated surgical system **1000** to affect the surgical volume **900** of ocular tissue shown in FIG. 11, and a two-dimensional schematic illustration of the treatment pattern **P1** overlaying anatomical structures to be treated. FIG. 12b is essentially the same as FIG. 12a, but more clearly illustrates an orthogonal relationship between the treatment pattern **P1** and the laser beam **701.** FIG. 13 is a three-dimensional schematic illustration of the anatomical structures of the eye including an opening **902** through the that results from the application of the laser treatment pattern of FIGS. 12a and 12b. The opening **902** provides and outflow pathway **40** that reduces the flow resistance in the ocular tissue to increase aqueous flow from the anterior chamber **7** into the Schlemm's canal **18** and thereby reduce the IOP of the eye.

Surgical treatments reduce outflow pathway resistance while minimizing ocular tissue modification through design and selection of laser treatment patterns. A treatment pattern is considered to define a collection of a laser-tissue interaction volumes, referred to herein as cells. The size of a cell is determined by the extent of the influence of the laser-tissue interaction. When the laser spots, or cells, are spaced close along a line, the laser creates a narrow, microscopic channel. A wider channel can be created by closely spacing a multitude of laser spots within the cross section of the channel. The arrangement of the cells may resemble the arrangement of atoms in a crystal structure.

With reference to FIGS. 12a and 12b, a treatment pattern **P1** may be in the form of a cubic structure that encompasses individual cells arranged in regularly spaced rows, columns and sheets or layers. The treatment pattern **P1** may be characterized by x, y, z dimensions, with x, y, z coordinates of the cells being calculated sequentially from neighbor to neighbor in the order of a column location (x coordinate), a row location (y coordinate), and a layer location (z coordinate). A treatment pattern **P1** as such, defines a three-dimensional model of ocular tissue to be modified by a laser or a three-dimensional model of ocular fluid to be affected by a laser.

A treatment pattern **P1** is typically defined by a set of surgical parameters. The surgical parameters may include one or more of a treatment area *A* that represents a surface area or layer of ocular tissue through which the laser will travel. The treatment area *A* is determined by the treatment height, *h*, and the lateral extent of the treatment, *w*. A treatment thickness *t* that represents the level to which the laser will cut into the ocular tissue from the distal extent or border of the treatment volume at or near Schlemm's canal **18** to the proximal extent or border at or near the surface of the trabecular meshwork **12.** Thus, a laser applied in accordance with a treatment pattern may affect or produce a surgical volume that resembles the three-dimensional model of the treatment pattern, or may affect fluid located in an interior of an eye structure resembled by the three-dimensional model.

Additional surgical parameters define the placement of the surgical volume or affected volume within the eye. For example, with reference to FIGS. 11, 12a, and 12b, placement parameters may include one or more of a location *l* that represents where the treatment is to occur relative to the circumferential angle of the eye, and a treatment depth *d* that represents a position of the three-dimensional model of ocular tissue or ocular fluid within the eye relative to a reference eye structure. In the following, the treatment depth *d* is shown and described relative to the region where the anterior chamber **7** meets the trabecular meshwork **12.** Together, the treatment pattern and the placement parameters define a treatment plan.

A femtosecond laser provides highly localized, non-thermal photo-disruptive laser-tissue interaction with minimal collateral damage to surrounding ocular tissue. Photo-disruptive interaction of the laser is utilized in optically transparent tissue. The principal mechanism of laser energy deposition into the ocular tissue is not by absorption but by a highly nonlinear multiphoton process. This process is effective only at the focus of the pulsed laser where the peak intensity is high. Regions where the beam is traversed but not at the focus are not affected by the laser. Therefore, the interaction region with the ocular tissue is highly localized both transversally and axially along the laser beam.

With reference to FIGS. 11, 12a, and 12b, in accordance with embodiments disclosed herein a surgical volume **900** of ocular tissue to be treated is identified by the integrated surgical system **1000** and a treatment pattern **P1** corresponding to the surgical volume is designed by the integrated surgical system. Alternatively, the treatment pattern **P1** may be designed first, and then an appropriate surgical volume **900** for applying the treatment pattern may be identified. The surgical volume **900** of ocular tissue may comprise portions of the trabecular meshwork **12** and the Schlemm's canal **18.** For example, the surgical volume **900** of ocular tissue shown in FIG. 11 includes portions of the uveal **15,** the corneoscleral meshwork **16,** the juxtacanalicular tissue **17,** and the inner **wall 18a** of the Schlemm's canal **18.** The treatment pattern **P1** defines a laser scanning procedure whereby a laser is focused at different depth locations in ocular tissue and then scanned in multiple directions to affect a three-dimensional volume of tissue comprising multiple sheets or layers of affected tissue.

With reference to FIGS. 12a, 12b, and 13, during a laser scanning procedure, a surgical laser **701** may scan ocular tissue in accordance with the treatment pattern **P1** to form an opening **902** that extends from the anterior chamber **7,** through each of the uveal **15,** the corneoscleral meshwork **16,** the juxtacanalicular tissue **17** of the trabecular meshwork **12,** and the inner wall **18a** of the Schlemm's canal **18.** While the example opening **902** in FIG. 13 is depicted as a continuous, single lumen defining a fluid pathway, the opening may be defined an arrangement of adjacent pores forming a sponge like structure defining a fluid pathway or a combination thereof. While the example opening **902** in FIG. 13 is in the shape of a cube, the opening may have other geometric shapes.

The movement of the laser as it scans to affect the surgical volume **900** follows the treatment pattern **P1**, which is defined by a set of surgical parameters that include a treatment area *A* and a thickness *t.* The treatment area *A* is defined by a width *w* and a height *h*. The width may be defined in terms of a measure around the circumferential angle. For example, the width w may be defined in terms of an angle, e.g., 90 degrees, around the circumferential angle.

Referring to FIGS. 11, 12a, and 12b, an initial placement of the laser focus within the eye is defined by a set of placement parameters, including a depth *d* and a location *l.* The location *l* defines a point around the circumferential angle of the eye at which laser treatment will begin, while the depth *d* defines a point between the anterior chamber **7** and the Schlemm's canal **18** where the laser treatment begins or ends. The depth *d* is measured relative to the region where the anterior chamber **7** meets the trabecular meshwork **12.** Thus, a first point that is closer to the Schlemm's canal **18** side of the trabecular meshwork **12** may be described as being deeper than a second point that is closer to the anterior chamber **7** side of the trabecular meshwork **12.** Alternatively, the second point may be described as being shallower than the first point.

With reference to FIG. 13, the opening **902** resulting from laser application of the treatment pattern **P1** resembles the surgical volume **900** and is characterized by an area *A* and thickness *t* similar to those of the surgical volume and the treatment pattern. The thickness *t* of the resulting opening **902** extends from the anterior chamber **7** and through the inner wall **18a** of the Schlemm's canal **18,** while the area *A* defines the cross-section size of the opening **902.**

In accordance with embodiments disclosed herein, during a laser scanning procedure, a laser focus is moved to different depths *d* in ocular tissue and then scanned in two lateral dimensions or directions as defined by a treatment pattern **P1** to affect a three-dimensional volume **900** of ocular tissue comprising multiple sheets or layers of affected tissue. The two lateral dimensions are generally orthogonal to the axis of movement of the laser focus. With reference to FIG. 13, the movement of a laser focus during laser scanning is described herein with reference to x, y, and z directions or axes, wherein: 1) movement of the laser focus to different depths *d* through the thickness *t* of treatment pattern **P1** or the volume **900** of tissue corresponds to movement of the focus along the z axis, 2) movement of the laser focus in two dimensions or directions orthogonal to the z axis corresponds to movement of the laser focus along the width w of the treatment pattern **P1** or the volume **900** of tissue in the x direction, and movement of the laser focus along the height *h* of the treatment pattern **P1** or the volume **900** of tissue in the y direction.

As used herein scanning of the laser focus generally corresponds to a raster type movement of the laser focus in the x direction, the y direction, and the z direction. The laser focus may be located at a point in the z direction and then raster scanned in two dimensions or directions, in the x direction and the y direction. The focal point of the laser in the z direction may be referred to as a depth *d* within the treatment pattern **P1** or the volume **900** of tissue. The two direction raster scanning of the laser focus defines a layer of laser scanning, which in turn produces a layer of laser-affected tissue.

During laser scanning, pulse shots of a laser are delivered to tissue within the volume of ocular tissue corresponding to the treatment pattern **P1**. Because the laser interaction volume is small, on the order of a few micrometers (µm), the interaction of ocular tissue with each laser shot of a repetitive laser breaks down ocular tissue locally at the focus of the laser. Pulse duration of the laser for photo-disruptive interaction in ocular tissue can range from several femtoseconds to several nanoseconds and pulse energies from several nanojoules to tens of microjoules. The laser pulses at the focus, through multiphoton processes, breaks down chemical bonds in the molecules, locally photo-dissociate tissue material and create gas bubbles in wet tissue. The breakdown of tissue material and mechanical stress from bubble formation fragments the tissue and create clean continuous cuts when the laser pulses are laid down in proximity to one another along geometrical lines and surfaces.

Table 2 includes examples of treatment pattern parameters and surgical laser parameters for treating tissue. The range of the parameter set is limited by practical ranges for the repetition rate of the laser and the scanning speed of the scanners.

**Table 2**

| Tissue treated | Treatment pattern dimensions *w*[mm], *h*[mm], *t*[mm] | Opening cross section *A* [mm²] | Cell size *w*[µm], *h*[µm], *t*[µm] | Laser average power [W] | Laser repetition rate [kHz] | Laser pulse energy [µJ] | Procedure time [s] |
|---|---|---|---|---|---|---|---|
| Trabecular meshwork | 1.5, 0.2, 0.2 | 0.3 | 3, 3, 3 | 0.9 | 300 | 3 | 7.4 |
| Trabecular meshwork | 2, 0.2, 0.2 | 0.4 | 4, 4, 4 | 1 | 200 | 5 | 6.3 |

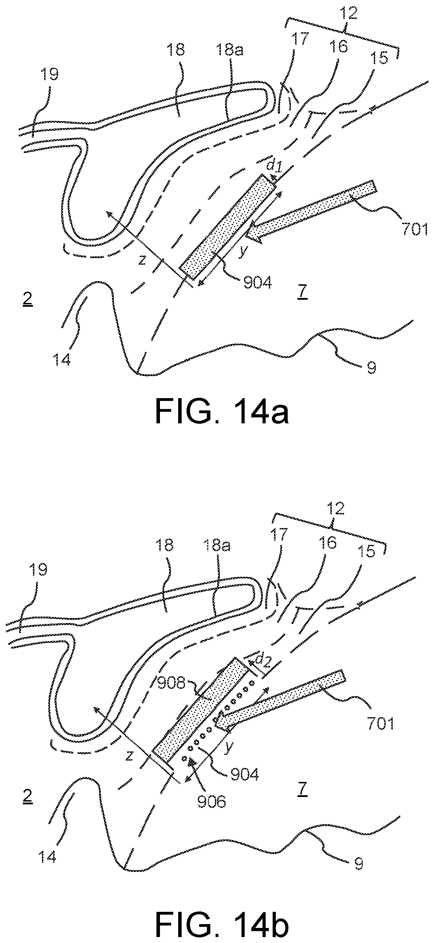

With reference to FIGS. 11, 12a, 12b, 13, 14a and 14b, in one type of laser scanning procedure, the scanning begins at the end of the treatment pattern **P1** adjacent the anterior chamber **7** and proceeds in a direction that generally corresponds to the direction of propagation of the laser **701.** More specifically, and with reference to FIG. 14a, the laser scanning proceeds in the z direction toward an anatomical structure, e.g., the inner wall **18a** of the Schlemm's canal **18,** while the direction of propagation of the laser **701** also proceeds toward same anatomical structure, e.g., the inner wall **18a** of the Schlemm's canal **18.**

Laser scanning in this manner, however, may be ineffective at producing the desired opening **902** between the anterior chamber **7** and the Schlemm's canal **18** due to interference by gas bubbles produced during laser application. As noted above, femtosecond lasers generate a very short pulse of optical energy. When a beam of such pulses is focused to a very small volume of space characterized by a small cross-sectional area, a non-linear effect occurs within the focus spot. When such a focus spot is directed onto tissue, the tissue is photodisrupted (broken down) leaving a small bubble of gas. This process is essentially non-thermal and requires a tiny amount of energy. The result is that the surrounding tissue is not affected.

However, when a femtosecond laser beam is scanned over the surface of a tissue, the laser treatment of this initial surface layer generates a layer of bubbles over the area of the treatment. When the laser scans the layer of tissue below or deeper than the initial surface layer, these bubbles create a shadow effect that scatters the incident laser light, effectively blocking further treatment of the tissue. This renders further laser treatment of tissue beneath or deeper that the initial surface layer ineffective.

An example of this effect within the context of glaucoma surgery is illustrated in FIGS. 14a and 14b. In FIG 14a, the focus of the laser beam **701** is initially located at a depth *d₁.* This depth *d₁* places the laser focus in an initial layer **904** of tissue. For example, initial layer **904** of tissue may be at the interface between the uveal **15** of the trabecular meshwork **12** and the anterior chamber **7.** In this instance, this depth location of the laser focus is referred to a null depth and the initial layer **904** to be treated corresponds to the surface of the uveal **15** facing the anterior chamber **7.** Once the laser focus is positioned at the initial depth *d₁,* the focus is scanned in multiple directions while being maintained at the initial depth. With reference to FIG. 14a, the multiple directions are the x direction and y direction, where the x direction is into the plane of FIG. 14a.

With reference to FIG. 14b, the raster scanning in the multiple directions results in the photodisruption of the initial layer **904** of tissue and the formation of a layer of bubbles **906** at the initial layer of tissue. The focus of the laser beam **701** is then moved in the z direction toward the inner **wall 18a** of the Schlemm's canal **18** to another depth *d₂.* This depth *d₂* places the laser focus at a subsequent layer **908** of tissue deeper than the initial layer **904.** For example, the deeper layer of tissue may comprise the uveal **15** of the trabecular meshwork **12.** Once the laser focus is positioned at the subsequent layer **908,** the focus is raster scanned in multiple directions while being maintained at that depth. However, in this instance, the layer of bubbles **906** scatters the incident laser light, effectively blocking further treatment of the tissue at the subsequent layer **908.**

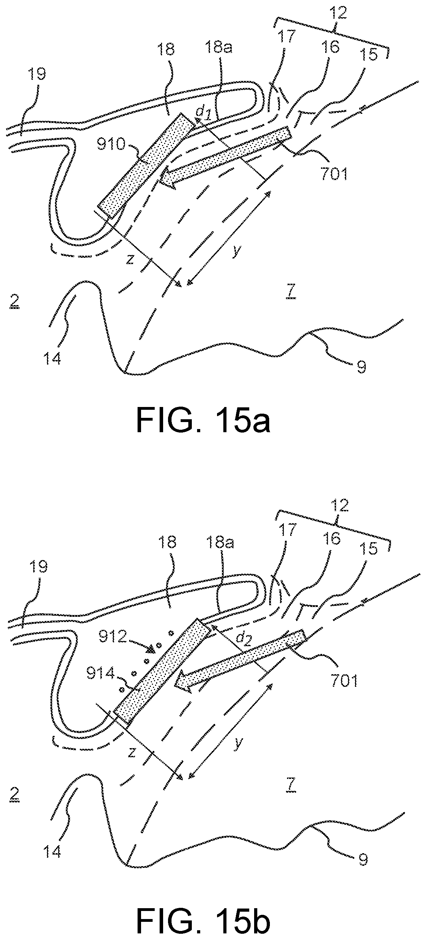

With reference to FIGS. 11, 12a, 12b, 13, 15a-15g, in accordance with embodiments disclosed the above ineffective laser treatment is avoided by implementing a laser scanning procedure, whereby the laser scanning begins at the end of the treatment pattern **P1** adjacent the Schlemm's canal **18** and proceeds in a direction generally opposite to or against the direction of propagation of the laser **701.** More specifically, and with reference to FIG. 15a, the laser scanning starts at an anatomical structure, e.g., the inner wall **18a** of the Schlemm's canal **18** and proceeds away from that structure in the z direction toward the anterior chamber **7,** while the direction of propagation of the laser **701** proceeds toward the that structure.

With this scanning procedure, the laser beam of femtosecond pulses is focused within a volume of ocular tissue at an initial depth or distance from a surface of the volume of tissue. An initial layer of tissue at the initial depth is treated, which generates a layer of bubbles at the area of the initial layer. After treatment of the initial layer of tissue, the laser is refocused to a subsequent layer of tissue that is shallower than the initial layer of tissue, i.e., at a depth that is closer to the surface of the volume of ocular tissue than the initial depth. Since the layer of bubbles at the area of the initial layer is below the second layer, the bubbles do not obstruct the second layer. This process is repeated until the laser scans, layer-by-layer through the volume of ocular tissue to the surface of the volume of tissue.

An example of this scanning procedure within the context of glaucoma surgery is illustrated in FIGS. 15a-15g. In FIG. 15a, the focus of the laser beam **701** is initially located at a depth *d₁.* This depth *d₁* places the laser focus in an initial layer **910** of tissue. For example, initial layer **910** of tissue may comprise the inner wall **18a** of the Schlemm's canal **18.** Once the laser focus is positioned at the initial depth *d₁,* the focus is scanned in multiple directions while being maintained at the initial depth *d₁.* With reference to FIG. 15a, the multiple directions are the x direction and y direction, where the x direction is into the plane of FIG. 15a.

With reference to FIG. 15b, the laser scanning in multiple directions results in the photodisruption of the initial layer **910** of tissue and the formation of a layer of gas bubbles **912** at the location of the initial layer of tissue. The focus of the laser beam **701** is then moved in the z direction toward the anterior chamber **7** to a subsequent depth *d₂.* The subsequent depth *d₂* places the laser focus at a subsequent layer **914** of tissue less deep than the initial layer **910** of tissue. For example, the subsequent layer **914** of tissue may comprise a portion of the inner wall **18a** of the Schlemm's canal **18,** the juxtacanalicular tissue **17,** and the corneoscleral meshwork **16.** Once the laser focus is positioned at the subsequent depth *d₂,* the focus is scanned in multiple directions while being maintained at the subsequent depth *d₂.* Since the layer of gas bubbles **912** is beneath the subsequent layer **914,** the bubbles do not obstruct laser access to or block photodisruption of the subsequent layer.

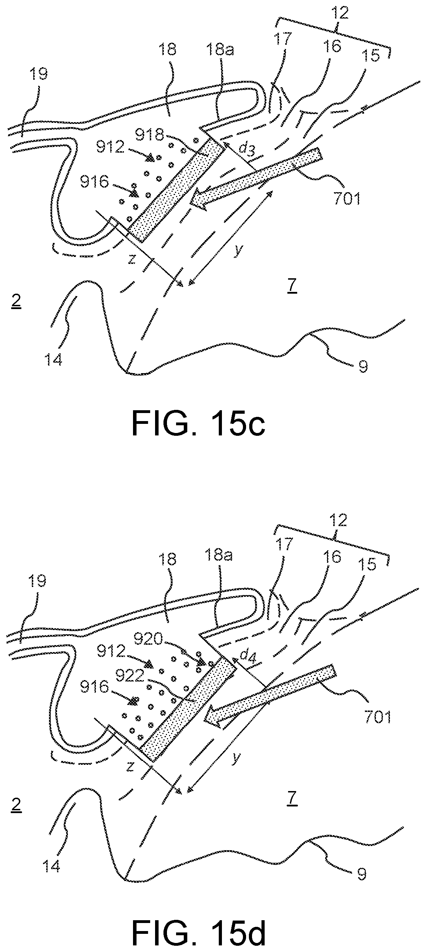

With reference to FIG. 15c, the laser scanning in multiple directions results in the photodisruption of the subsequent layer **914** of tissue and the formation of a layer of bubbles **916** at the location of the subsequent layer of tissue. The focus of the laser beam **701** is then moved in the z direction toward the anterior chamber **7** to a subsequent depth *d₃.* The subsequent depth *d₃* places the laser focus at a subsequent layer **918** of tissue less deep than the subsequent layer **914** of tissue. For example, the subsequent layer **914** of tissue may comprise a portion of the juxtacanalicular tissue **17** and the corneoscleral meshwork **16.** Once the laser focus is positioned at the subsequent depth *d₃,* the focus is scanned in multiple directions while being maintained at the subsequent depth *d₃.* Since the layers of gas bubbles **912, 916** are beneath the subsequent layer **918,** the bubbles do not obstruct laser access to or block photodisruption of the subsequent layer.

With reference to FIG. 15d, the laser scanning in multiple directions results in the photodisruption of the subsequent layer **918** of tissue and the formation of a layer of bubbles **920** at the location of the subsequent layer of tissue. The focus of the laser beam **701** is then moved in the z direction toward the anterior chamber **7** to a subsequent depth *d₄.* The subsequent depth *d₄* places the laser focus at a subsequent layer **922** of tissue less deep than the subsequent layer **918** of tissue. For example, the subsequent layer **922** of tissue may comprise a portion of the corneoscleral meshwork **16** and the uveal **15.** Once the laser focus is positioned at the subsequent depth *d₄,* the focus is scanned in multiple directions while being maintained at the subsequent depth *d₄*. Since the layers of gas bubbles **912, 916, 920** are beneath the subsequent layer **922,** the bubbles do not obstruct laser access to or block photodisruption of the subsequent layer.

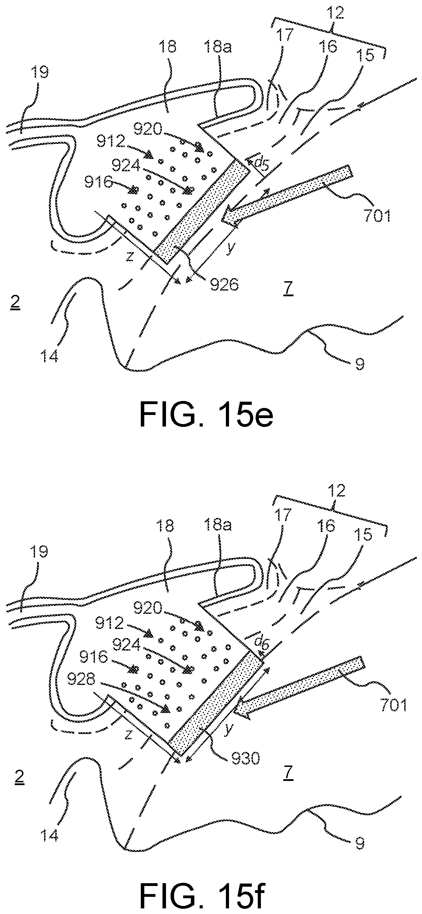

With reference to FIG. 15e, the laser scanning in multiple directions results in the photodisruption of the subsequent layer **922** of tissue and the formation of a layer of bubbles **924** at the location of the subsequent layer of tissue. The focus of the laser beam **701** is then moved in the z direction toward the anterior chamber **7** to a subsequent depth *d₅.* The subsequent depth *d₅* places the laser focus at a subsequent layer **926** of tissue less deep than the subsequent layer **922** of tissue. For example, the subsequent layer **926** of tissue may comprise the uveal **15.** Once the laser focus is positioned at the subsequent depth *d₅,* the focus is scanned in multiple directions while being maintained at the subsequent depth *d₅.* Since the layers of gas bubbles **912, 916, 920, 924** are beneath the subsequent layer **926,** the bubbles do not obstruct laser access to or block photodisruption of the subsequent layer.

With reference to FIG. 15f, the laser scanning in multiple directions results in the photodisruption of the subsequent layer **926** of tissue and the formation of a layer of bubbles **928** at the location of the subsequent layer of tissue. The focus of the laser beam **701** is then moved in the z direction toward the anterior chamber **7** to a subsequent depth *d₆.* The subsequent depth *d₆* places the laser focus at a subsequent layer **930** of tissue less deep than the subsequent layer **926** of tissue. For example, the subsequent layer **930** of tissue may comprise the uveal **15** and the inner surface of the uveal facing the anterior chamber **7.** Once the laser focus is positioned at the subsequent depth *d₆,* the focus is scanned in multiple directions while being maintained at the subsequent depth *d₆.* Since the layers of gas bubbles **912, 916, 920, 924, 928** are beneath the subsequent layer **930,** the bubbles do not obstruct laser access to or block photodisruption of the subsequent layer.

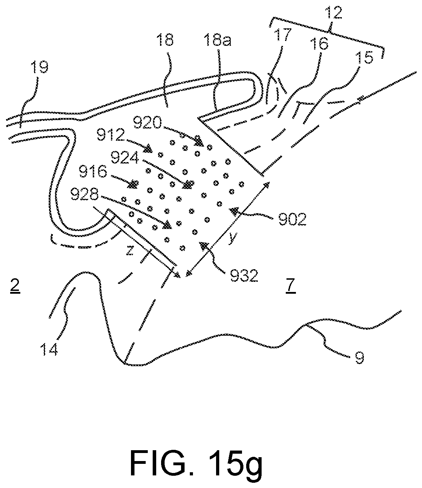

With reference to FIG. 15g, the laser scanning in multiple directions results in the photodisruption of the subsequent layer **930** of tissue and the formation of a layer of bubbles **932** at the location of the subsequent layer of tissue. Photodisruption of this subsequent layer **930** of tissue results in the formation of an opening **902** between the anterior chamber **7** and the Schlemm's canal **18,** thus completing the laser treatment procedure.

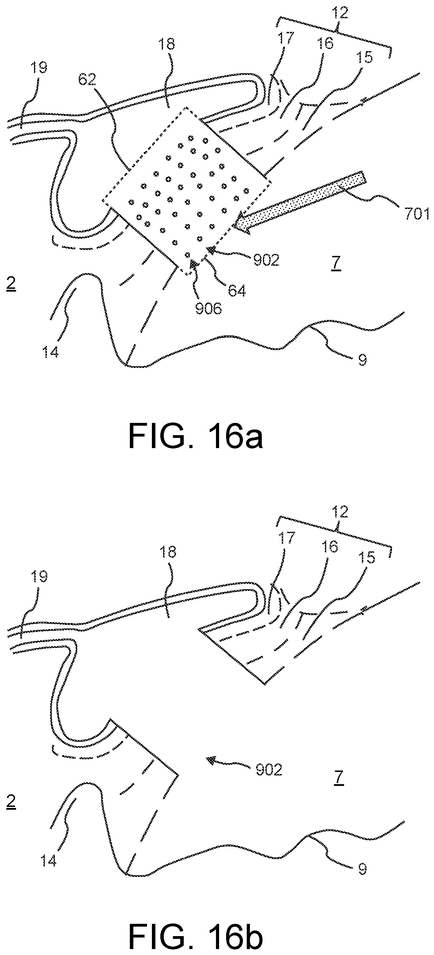

With reference to FIG. 16a, upon completion of the laser scanning the opening **902** may be partially obstructed or occluded by the gas bubbles **912, 916, 920, 924, 928** created during treatment. Thus, in accordance with embodiments disclosed herein, the direction of the laser scanning described with reference to FIGS. 15a-15g may be reversed in order to push any remaining bubbles into the Schlemm's canal **18** thereby clearing the opening **902,** as shown in FIG. 16b.

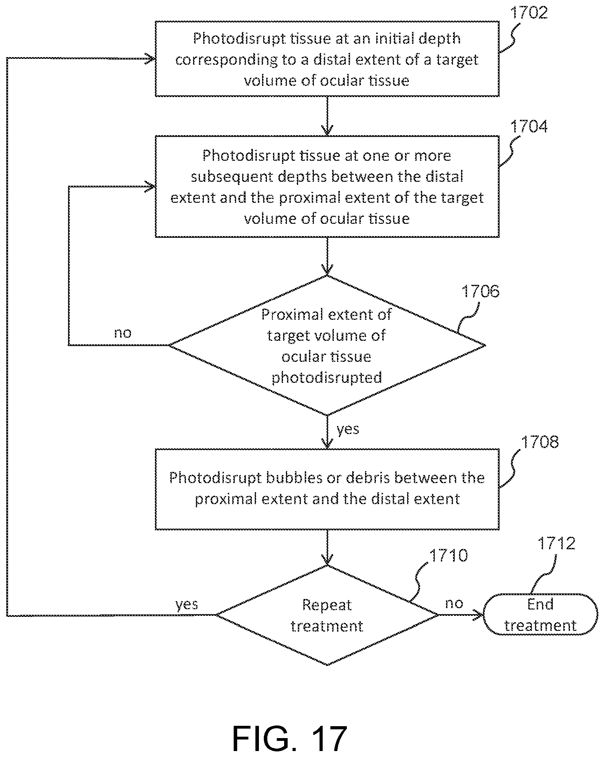

FIG. 17 is a flowchart of a method of treating a target volume of ocular tissue with a laser having a direction of propagation toward the target volume of ocular tissue. With reference to FIGS. 12a and 12b, the target volume **60** of ocular tissue is characterized by a distal extent **62,** a proximal extent **64,** and a lateral extent **66.** The distal extent **62** corresponds to the part or point of the target volume **60** that is most distal along the direction of propagation of the laser **701.** The proximal extent **64** corresponds to the part or point of the target volume **60** that is most proximal along the direction of propagation of the laser **701.** The lateral extent **66** corresponds to the distance or width *w* of the target volume **60** along the circumference angle.

The method, which may be performed by the integrated surgical system **1000** of FIGS. 7-10b, begins at a point in a surgical procedure where access to the irido-corneal angle has already been obtained and the target volume **60** of ocular tissue has already been identified for treatment. Systems and methods for accessing the irido-corneal angle are described in U.S. Patent Application Serial No. 16/036,883, entitled Integrated Surgical System and Method for Treatment in the Irido-Corneal Angle of the Eye. Systems and method for identifying volumes of ocular tissue for treatment and designing treatment patterns reference are described in U.S. Patent Application Serial No. 16/125,588, entitled Non-Invasive and Minimally Invasive Laser Surgery for the Reduction of Intraocular Pressure in the Eye.

At block **1702,** the integrated surgical system **1000** initially photodisrupts tissue at an initial depth *d₁* corresponding to the distal extent **62** of the target volume **60** of ocular tissue. To this end, and with reference to FIG. 15a, the integrated surgical system **1000** focuses light from a femtosecond laser **701** at a spot in the tissue at the initial depth *d₁* and applies optical energy to the tissue, which energy is at a level sufficient to photodisrupt the tissue. Optical energy is applied by scanning the laser **701** in multiple directions defining an initial treatment plane **910** at the initial depth *d₁* to thereby photodisrupt an initial layer of tissue of the target volume of ocular tissue. With reference to FIG. 13, the scanning may be in the form of a raster scan where the laser is scanned in a first direction along the lateral extent **66,** i.e., the x direction, and then slightly repositioned in a second direction. i.e., the y direction, and then scanned again along the lateral extent.

As an additional aspect of the initial photodisruption process of block **1702,** the integrated surgical system **1000** may detect the distal extent **62** of the target volume of ocular tissue. To this end, in one configuration images captured by the OCT imaging apparatus **300** are processed by the control system **100** to detect the distal extent **62** of the target volume using known techniques. In another configuration, the integrated surgical system **1000** may include a multiphoton imaging apparatus (not shown) that provides a visual indication on a display of the user interface **110** that is indicative of the location of the focus of the laser **701** relative to the distal extent **62** of the target volume **60** of ocular tissue. The integrated surgical system **1000** may also determine the lateral extent **66** of the target volume **60** of ocular tissue based on OCT imaging.

At block **1704** and with reference to FIGS. 15b-15f, the integrated surgical system **1000** subsequently photodisrupts tissue at one or more subsequent depths *d₂-d₆* between the distal extent **62** of the target volume **60** of ocular tissue and the proximal extent **64** of the target volume of ocular tissue is by moving a focus of the laser **701** in a direction opposite the direction of propagation of the laser. To this end, the integrated surgical system **1000** focuses light from a femtosecond laser **701** at a spot in the tissue at the one or more subsequent depths *d₂-d₆* and applies optical energy to the tissue, which energy is at a level sufficient to photodisrupt the tissue. Optical energy is applied by scanning the laser **701** in multiple directions defining a subsequent treatment plane **914, 918, 922, 926, 930** at a respective different depth *d₂-d₆,* to thereby photodisrupt one or more subsequent layers of tissue of the target volume **60** of ocular tissue. With reference to FIG. 13, the scanning may be in the form of a raster scan where the laser is scanned in a first direction along the lateral extent **66,** i.e., the x direction, and then slightly repositioned in a second direction. i.e., the y direction, and then scanned again along the lateral extent.

As an additional aspect of the subsequent photodisruption process of block **1704,** the integrated surgical system **1000** may detect the proximal extent **64** of the target volume **60** of ocular tissue. To this end, in one configuration images captured by the OCT imaging apparatus **300** are processed by the control system **100** to detect the proximal extent **64** of the target volume **60** using known techniques. In another configuration, the integrated surgical system **1000** may include a multiphoton imaging apparatus (not shown) that provides a visual indication on a display of the user interface **110** that is indicative of the location of the focus of the laser **701** relative to the proximal extent **64** of the target volume **60** of ocular tissue. In yet another configuration, the integrated surgical system **1000** may include an opto-mechanical imaging apparatus (not shown) that provides a visual indication on a display of the user interface **110** that is indicative of the location of the focus of the laser **701** relative to the proximal extent **64** of the target volume **60** of ocular tissue.

At block **1706,** the integrated surgical system **1000** determines if the proximal extent **64** of the target volume **60** of ocular tissue has been photodisrupted. If the proximal extent **64** has not been photodisrupted, the process return to block **1704** and the integrated surgical system **1000** repeats the photodisrupting at one or more subsequent depths until tissue at the proximal extent **64** of the target volume **60** of ocular tissue is photodisrupted.

Returning to block **1706** and with reference to FIG. 16a, if the proximal extent **64** has been photodisrupted, the process proceeds to block **1708** and the integrated surgical system **1000** photodisrupts tissue debris or bubbles **906** between the proximal extent **64** of the target volume **60** of ocular tissue and the distal extent **62** of the target volume by moving the focus of the laser **701** in the direction of propagation of the laser. To this end, the integrated surgical system **1000** focuses light from a femtosecond laser **701** at a spot in the volume of tissue debris or bubbles **906** at the one or more subsequent depths and applies optical energy to the tissue debris or bubbles. Optical energy is applied by scanning the laser **701** in multiple directions along one or more of the previously-scanned treatment planes **910, 914, 918, 922, 926, 930** to photodisrupt tissue debris or bubbles **906** between the proximal extent **64** and the distal extent **62** of the photodisrupted target volume **60.**

Progression of clearing the debris and bubbles happens from the proximal extent **64** of the of the target volume **60** towards the distal extent **62** of the target volume. At the proximal boundary between target tissue, the trabecular meshwork and the aqueous humor gas bubbles adhere to the surface initially by forces of surface tension, preventing laser light from penetrating into more distant locations within the target volume **60.** On subsequent repeats of the scanning pattern the volume of the gas bubbles grows, increasing the buoyancy of the bubbles. As the buoyant forces overcome the surface tension forces, the gas bubbles free from the surface and rise up within the aqueous humor, similar to how gas bubbles sticked to the wall of a champagne glass grow and rise as the bubbles gets larger. In addition to the buoyant forces, dynamic forces from the creation of bubbles in the eye by the laser facilitate freeing the bubbles from the surface. Accordingly, during subsequent repeats of the treatment pattern the progression of the extent in the tissue is from proximal to distal, i.e., from depth *d₆* to depth *d₁.*

At block **1710,** the integrated surgical system **1000** may determine to repeat the treatment of the photodisrupted target volume **60** of ocular tissue or to end the treatment. If treatment is repeated, the process returns to block **1702,** where the integrated surgical system **1000** repeats the initial photodisrupting of tissue, and then proceeds to blocks **1704** and **1706,** where the system repeats the subsequent photodisrupting of tissue one or more times. If treatment is not to be repeated, the process proceeds to block **1712,** where treatment ends.

Regarding the use of a multiphoton imaging apparatus to detect the distal extent **62** of the target volume of ocular tissue, or the proximal extent **64** of the target volume, such an apparatus is configured to present an image of a second harmonic light that results from an encounter between the focus of the laser 701 and tissue. When the focus of the laser 701 is not encountering tissue, the intensity of the second harmonic light is zero or very low. When the focus is encountering tissue, the intensity of the second harmonic light increases. Based on this, a distal extent **62** such as shown in FIGS. 12a and 12b may be detected by first advancing the focus of the laser **701** through the trabecular meshwork **12** and the inner wall **18a** of the Schlemm's canal and into the Schlemm's canal **18,** where the focus will not encounter light and the intensity of the second harmonic light is zero or very low, and then retracting the focus back toward the inner wall **18a** of the Schlemm's canal and detecting that the focus is at the inner wall when an increase in the intensity of the second harmonic light is noted on the display.

Regarding the use of an opto-mechanical imaging apparatus to detect the proximal extent **64** of the target volume **60** of ocular tissue, such an apparatus is configured to direct a first beam of light and a second beam of light to be incident with the target volume and to align the first beam of light and the second beam of light relative to each other and relative to the laser beam such that the first beam of light and the second beam light intersect at a point corresponding to the focus of the laser. The apparatus is also configured to capture an image of a first spot corresponding to the first beam of light, and a second spot corresponding to the second beam of light relative to the proximal extent **64** of the target volume **60** of ocular tissue. The first and second spots appear in the image as two separate visible spots on the surface of the proximal extent **64** when the focus is away from the surface, and as a single, overlapping spot when the focus is on the surface. Accordingly, the proximal extent **64** is detected when the spots overlap.

With reference to FIGS. 7-10b, a surgical system **1000** for implementing the method of FIG. 17 includes a first optical subsystem **1001** including a focusing objective **700** configured to be coupled to the eye **1,** and a second optical subsystem **1002** including a laser source **200** configured to output a laser beam **201/701.** The second optical subsystem **1002** also includes a plurality of components **1003** configured to one or more of focus, scan, and direct the laser beam through the focusing objective, in a direction of propagation toward the target volume of ocular tissue.

The surgical system **1000** further includes a control system **100** coupled to the second optical subsystem **1002** and configured to control the focus and scan of the laser beam **701** to photodisrupt tissue at an initial depth corresponding to the distal extent of the target volume of ocular tissue. To this end, the control system **100** is configured to focus light from a femtosecond laser source **200** at a spot in the tissue at the initial depth and then apply optical energy to the tissue, where the energy is sufficient to photodisrupt tissue. The control system **100** controls the focus and scan of the laser beam **701** during application of optical energy by being further configured to scan the laser in multiple directions defining an initial treatment plane, to thereby photodisrupt an initial layer of tissue of the target volume of ocular tissue.

The control system **100** is also configured to control the focus and scan of the laser beam **701** to photodisrupt tissue at one or more subsequent depths between the distal extent of the target volume of ocular tissue and the proximal extent of the target volume of ocular tissue by moving a focus of the laser in a direction opposite the direction of propagation of the laser. To this end, the control system **100** is configured to focus light from a femtosecond laser source **200** at a spot in the tissue at a subsequent depth and then apply optical energy to the tissue, where the energy is sufficient to photodisrupt tissue. The control system **100** controls the focus and scan of the laser beam **701** during application of optical energy by being further configured to scan the laser in multiple directions defining a subsequent treatment plane, to thereby photodisrupt a subsequent layer of tissue of the target volume of ocular tissue.

The control system **100** is also configured to control the focus and scan of the laser beam **701** to photodisrupt tissue debris or bubbles between the proximal extent of the target volume of ocular tissue and the distal extent of the target volume by moving the focus of the laser in the direction of propagation of the laser, after photodisrupting the target volume of ocular tissue. The control system **100** is further configured to control the focus and scan of the laser beam **701** to repeat the initial photodisrupting of tissue and the subsequent photodisrupting of tissue one or more times.

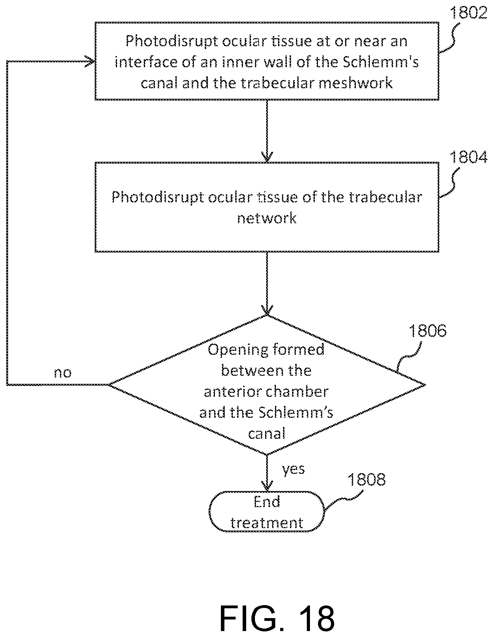

FIGS. 18 is a flowchart of a method of treating an eye comprising an anterior chamber, a Schlemm's canal, and a trabecular meshwork therebetween. The method, which may be performed by the integrated surgical system **1000** of FIGS. 7-10b, begins at a point in a surgical procedure where access to the irido-corneal angle has already been obtained and one or more anatomical structures of the eye that are to be treated have been located.

At block **1802** and with reference to FIGS. 15a and 15b, the integrated surgical system **1000** initially photodisrupts ocular tissue at or near an interface of an inner wall **18a** of the Schlemm's canal **18** and the trabecular meshwork **12.** To this end, the integrated surgical system **1000** focuses light from a femtosecond laser **701** at a spot in the ocular tissue at or near the interface of the inner wall **18a** of the Schlemm's canal **18** and the trabecular meshwork **12** and applies optical energy to the tissue, which energy is at a level sufficient to photodisrupt the tissue.

As an additional aspect of the initial photodisruption process of block **1802,** the integrated surgical system **1000** may detect ocular tissue at or near the interface of the inner wall **18a** of the Schlemm's canal **18** and the trabecular meshwork **12.** To this end, in one configuration images captured by the OCT imaging apparatus **300** are processed by the control system **100** to detect the interface of the inner wall **18a** of the Schlemm's canal **18** and the trabecular meshwork **12** using known techniques. In another configuration, the integrated surgical system **1000** may include a multiphoton imaging apparatus (not shown) that provides a visual indication on a display of the user interface **110** that is indicative of the location of the focus of the laser **701** relative to the interface of the inner **wall 18a** of the Schlemm's canal **18** and the trabecular meshwork **12.** The integrated surgical system **1000** may also determine a lateral extent **66** of ocular tissue to be photodisrupted based on OCT imaging.

At block **1804** and with reference to FIGS. 15c-15f, the integrated surgical system **1000** subsequently photodisrupts ocular tissue of the trabecular meshwork **12.** To this end, the integrated surgical system **1000** focuses light from a femtosecond laser **701** at a spot in tissue of the trabecular meshwork **12** and applies optical energy to the tissue, which energy is at a level sufficient to photodisrupt the tissue.

As an additional aspect of the subsequent photodisruption process of block **1804,** the integrated surgical system **1000** may detect a proximal extent of tissue of the trabecular meshwork. To this end, in one configuration images captured by the OCT imaging apparatus **300** are processed by the control system **100** to detect the proximal extent **64** of the tissue of the trabecular meshwork using known techniques. In another configuration, the integrated surgical system **1000** may include a multiphoton imaging apparatus (not shown) that provides a visual indication on a display of the user interface **110** that is indicative of the location of the focus of the laser **701** relative to the proximal extent **64** of the tissue of the trabecular meshwork. In yet another configuration, the integrated surgical system **1000** may include an opto-mechanical imaging apparatus (not shown) that provides a visual indication on a display of the user interface **110** that is indicative of the location of the focus of the laser **701** relative to the proximal extent **64** of the tissue of the trabecular meshwork.

At block **1806,** the integrated surgical system **1000** determines if an opening is formed between the anterior chamber and the Schlemm's canal. If an opening has not been formed, the process return to block **1802** and the integrated surgical system **1000** repeats the initial photodisrupting of ocular tissue and then proceeds to block **1804** and repeats the subsequent photodisrupting of ocular tissue one or more times until an opening is formed between the anterior chamber and the Schlemm's canal. If an opening has been formed, the process proceeds to block **1808,** where treatment ends.

With reference to FIGS. 7-10b, a system **1000** for implementing the method of FIG. 18 includes a first optical subsystem **1001** including a focusing objective **700** configured to be coupled to the eye **1,** and a second optical subsystem **1002** including a laser source **200** configured to output a laser beam **201/701.** The second optical subsystem **1002** also includes a plurality of components **1003** configured to one or more of focus, scan, and direct the laser beam through the focusing objective, toward ocular tissue.

The surgical system **1000** further includes a control system **100** coupled to the second optical subsystem **1002** and configured to control the focus and scan of the laser beam **701** to initially photodisrupt ocular tissue at or near an interface of an inner wall of the Schlemm's canal and the trabecular meshwork. To this end, the control system **100** is configured to focus light from a femtosecond laser source **200** at a spot in the ocular tissue at or near the interface of the inner wall of the Schlemm's canal and the trabecular meshwork, and then apply optical to the tissue, where the energy is sufficient to photodisrupt tissue.

The control system **100** is also configured to control the focus and scan of the laser beam **701** to subsequently photodisrupt tissue of the trabecular meshwork. To this end, the control system **100** is configured to focus light from a femtosecond laser at a spot in tissue of the trabecular meshwork, and then apply optical energy to the tissue, where the energy is sufficient to photodisrupt tissue. The control system **100** is further configured to control the focus and scan of the laser beam **701** to repeat the initial photodisrupting of ocular tissue and the subsequent photodisrupting of ocular tissue one or more times until an opening is formed between the anterior chamber and the Schlemm's canal.

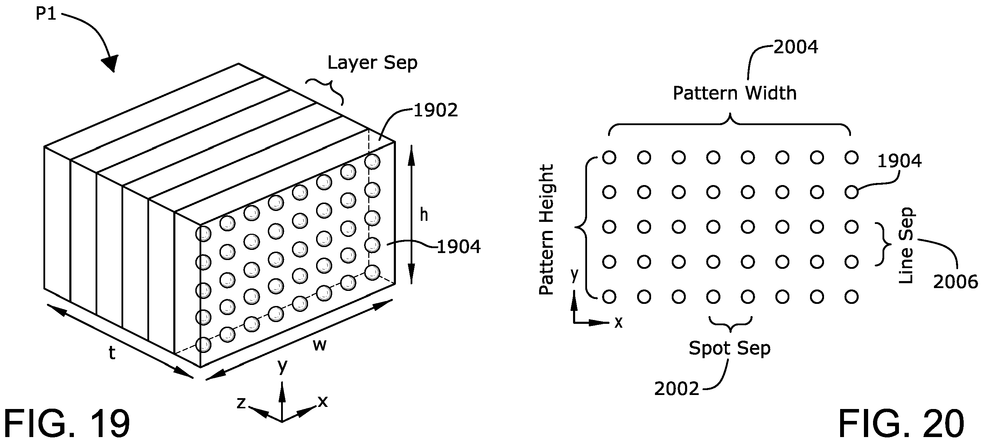

With reference to FIGS. 19 and 20, as previously described, a 3D treatment pattern **P1** may be defined by a number of 2D treatment layers **1902** or treatment planes that are stacked to form a 3D treatment pattern characterized by a width *w*, height *h*, and depth or thickness *t*. Each individual treatment layer **1902** is in turn characterized by a pattern height *h* (equal to the height *h* of the 3D treatment pattern **P1**) and a pattern width *w* (equal to the width *w* of the 3D treatment pattern **P1**) and comprises an array of spots **1904** spaced apart to establish or fit within the height and width. The pattern width *w* corresponds to a distance along the circumference of the corneal angle parallel to the trabecular meshwork. This direction is also known as the circumferential direction. The pattern height *h* corresponds to a distance transverse to the circumference of the corneal angle perpendicular to the trabecular meshwork. This direction is also known as the azimuthal direction.

Each spot **1904** in the treatment pattern **P1** corresponds to a site within a target volume of ocular tissue where optical energy is applied at a laser focus to create a micro-photodisruption site. With reference to FIG. 20, each spot **1904** in a treatment layer **1902** is separated from a neighboring spot by programmable distances called spot separation (Spot Sep) and a line separation (Line Sep). A treatment layer **1902** is completed with the programmed pattern width *w* and pattern height *h* is achieved. Each treatment layer **1902** in the 3D treatment pattern **P1** is separated from a neighboring layer by a layer separation (Layer Sep).

A treatment pattern **P1** may be defined by a set of programmable parameters, such as shown in Table 3.

**Table 3**

| Parameter | Minimum | Maximum |
|---|---|---|
| width *w* | 10 µm | 2000 µm |
| height *h* | 10 µm | 2000 µm |
| depth/thickness *t* | 10 µm | 4000 µm |
| Spot Sep | 2 µm | 40 µm |
| Line Sep | 2 µm | 40 µm |
| Layer Sep | 2 µm | 200 µm |
| pulse energy | 0 µJ | 35 µJ |

Other, non-rectangular and more irregular treatment patterns can also be programmed and created in the tissue. These irregular patterns can still be decomposed to spots, lines, and layers and their extent characterized by width, height, and depth. Examples of irregular treatment patterns are described in U.S. Patent Application Serial No. 16/838,858, entitled Method, System, and Apparatus for Generating Three-Dimensional Treatment Patterns for Laser Surgery of Glaucoma.

In one example treatment pattern **P1**, the parameters are:
width = 750 µm
height = 250 µm
depth = 350 µm
spot separation = 10 µm
line separation = 10 µm
layer separation = 10 µm

In one embodiment of laser treatment, such as described above with reference to FIGS. 15a-15g, each treatment layer **1902** is individually created by scanning the laser focus in two dimensions, e.g., width and height, or X and Y, to the various spots **1904** defining the layer, while the focus is fixed at the third dimension, e.g., depth or Z. Once a treatment layer **1902** is created, the focus is moved in the depth or z direction and the next treatment layer in the stack is created. This process is repeated until all treatment layers **1902** in the 3D treatment pattern **P1** are created.

In another embodiment, instead of creating a treatment pattern **P1** one treatment layer **1902** at a time, the laser focus is scanned in three dimensions. For example, while the laser focus is being moved transversely through a height and/or width, e.g., in the x and/or y direction, the laser focus is also oscillated back and forth axially through a depth, e.g., in the z direction. The treatment pattern **P1** characterized by such scanning of the laser focus may be referred to as a "clearing pattern." Oscillation of the laser focus through the depth in the z direction occurs simultaneous with transverse movement of the laser focus in the x and y directions through what is referred to herein as an XY pattern. As the laser focus quickly traverses back and forth through the depth, the oscillating jets of bubbles at the focus are sufficient to clear any gas bubble or debris from within the treatment volume. This clearing of gas and bubble debris provides a clearer optical view of the target volume of optical tissue, and thus more effective penetration of laser photodisruption.

The scanning mechanism, e.g., the transverse scanning mirrors **530** and **532** in FIG. 8, that moves the laser focus in the x direction and y direction through an XY pattern is independent of the scanning mechanism, e.g., axial scanning lens **520** in FIG. 8, that moves the laser focus in the z direction. Therefore, the velocity at which the laser focus moves through the XY pattern is independent of the velocity at which the laser focus moves in z direction. The velocity through the XY pattern, referred to herein as the transverse-scanning velocity, may be greater than, less than, or equal to the velocity in the z direction, referred to herein as the axial-scanning velocity.

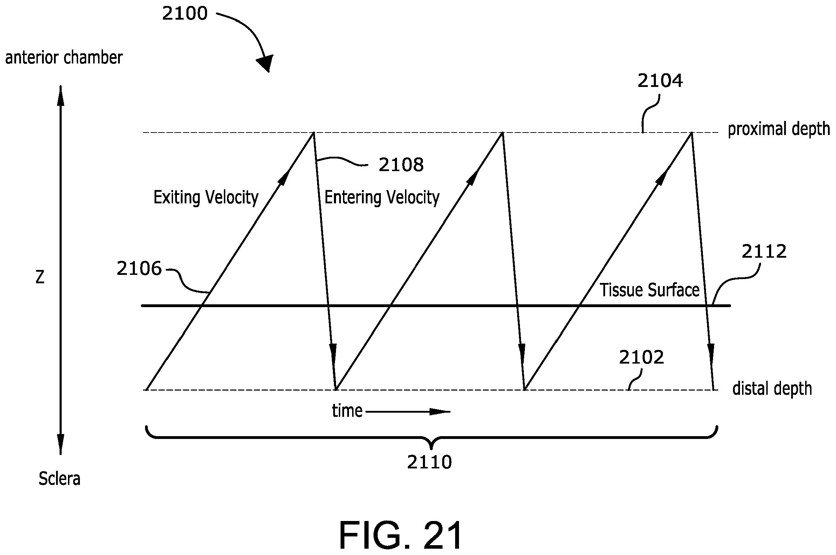

With reference to FIG. 21, movement of the laser focus in the z direction, while being scanned in three dimensions, may be characterized by a ramping profile **2100,** which in turn, may be defined by a set of clearing pattern parameters. The clearing pattern parameters include a distal depth **2102,** a proximal depth **2104,** an exiting velocity **2106,** an entering velocity **2108,** and a number of oscillations **2110.**

The distal depth **2102** corresponds to the distal extent **62** in FIGS. 12a and 12b and may be referenced or measured relative to a tissue surface **2112.** For example, the tissue surface **2112** may be the surface of the trabecular meshwork **12** facing the anterior chamber **7.** The distal depth **2102** may place the laser focus entirely in ocular tissue, or possibly outside of tissue and in another anatomical structure. For example, with reference to FIGS. 12a and 12b, an distal depth **2102** at the distal extent **62** may place the laser focus in ocular tissue such as the juxtacanalicular tissue **17** of the trabecular meshwork **12** or the inner wall of Schlemm's canal **18a,** or in another anatomical structure, e.g., the interior of the Schlemm's canal **18.**

The proximal depth **2104** corresponds to the proximal extent **64** in FIGS. 12a and 12b and may be referenced or measured relative to the tissue surface **2112.** For example, the tissue surface **2112** may be the surface of the trabecular meshwork **12** facing the anterior chamber **7.** The proximal depth **2104** may place the laser focus entirely in ocular tissue, or possibly outside of tissue and in another anatomical structure. For example, with reference to FIGS. 12a and 12b, an proximal depth **2104** at the proximal extent **64** may place the laser focus in ocular tissue such as the uveal **15** of the trabecular meshwork **12,** or in another anatomical structure, e.g., the anterior chamber **7.**

The exiting velocity **2106** specifies the speed at which the laser focus is moved between the distal depth **2102** and the proximal depth **2104.** The entering velocity **2108** specifies the speed at which the laser focus is moved between the proximal depth **2104** and the distal depth **2102.** The relationship between the exiting velocity **2106** and the entering velocity **2108** may be one where the velocities are the same, or one where the velocities are different. In one configuration, the entering velocity **2108** may be greater than the exiting velocity **2106.** For example, the entering velocity **2108** may be ten times greater than the exiting velocity **2106.**

Because the cutting action in photodisruption predominately occurs as the laser focus proceeds from the distal extent or distal depth **2102** to the proximal extent or proximal depth **2104,** the exiting velocity **2106** should be slow enough to assure an initial photodisruption of the tissues in the target volume of ocular tissue. The entering velocity **2108** should be rapid enough to push bubbles and debris out of the volume of ocular tissue. During each individual oscillation of the laser focus between the distal depth **2102** and the proximal depth **2104,** the exiting motion of the laser focus will further photodisrupt tissue within the volume of ocular tissue until the next entering motion.

The number of oscillations **2110** in a ramping profile **2100** corresponds to the number of times the laser focus is moved back and forth between the distal depth **2102** and the proximal depth **2104** during a period of time of a laser treatment. The number of oscillations **2110** in the example ramping profile **2100** of FIG. 21 is three. The total number of oscillations, however, may range from two to six-hundred. The period of time of the ramping profile **2100** may correspond to a time sufficient to photodisrupt enough spots of the volume of ocular tissue to create and aperture at the site of the volume.

While the ramping profile **2100** shown in FIG. 21 is characterized by a sawtooth shape, other shapes are contemplated. For example, the ramping profile may be any oscillatory pattern such as a sawtooth, a sine wave, a square wave, a triangle wave, or an arbitrary waveform. Note that the ramping profile need not be symmetric.

In one embodiment, movement of the laser focus through the XY pattern and movement of the laser focus in the z direction in accordance with a ramping profile **2100** are asynchronous. In other words, while laser focus movement through the XY pattern and the z direction using a ramping profile occur simultaneously, the speed of the respective movements are not synchronized to each other. As a result of this simultaneous and asynchronous movement, the laser focus under the influence of the XY pattern movement and the ramping profile in the z direction, meanders throughout a rectangle volume of ocular tissue to photodisrupt and clear the volume. As the number of times the laser focus is swept or scanned through the XY pattern, and the number of oscillations of the laser focus in the z direction increases, the extent that the meandering path of the laser focus "fills" the treatment volume increases.

A clearing pattern may be defined by a set of programmable parameters, such as shown in Table 4.

**Table 4**

| Parameter | Minimum | Maximum |
|---|---|---|
| proximal depth | 200µm | 3000µm |
| distal depth | 0µm | 200µm |
| exiting velocity | 50µm/s | 2000µm/s |
| entering velocity | 50µm/s | 6000µm/s |
| max oscillations | 2 | 600 |

In one example clearing pattern, the parameters may be:
proximal depth = 600µm
distal depth = 100µm
exiting velocity = 500 µm/s
entering velocity = 5000 µm/s
max oscillations = 50

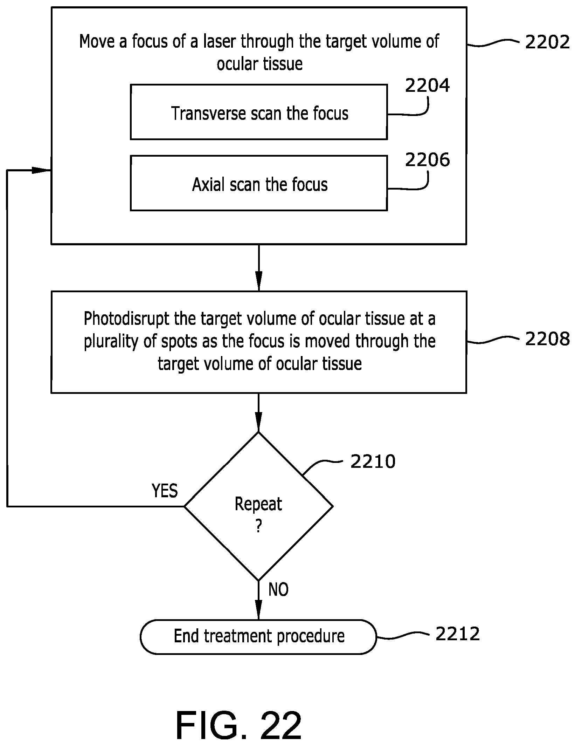

FIG. 22 is a flowchart of a method of treating a target volume of ocular tissue of an irido-corneal angle of an eye in accordance with a clearing pattern. The method, which may be performed by the integrated surgical system **1000** of FIGS. 7-10b, begins at a point in a surgical procedure where access to the irido-corneal angle has already been obtained and one or more anatomical structures of the eye that are to be treated have been located. The target volume of ocular tissue may be entirely within ocular tissue. Alternatively, at least a portion of the target volume of ocular tissue may encompass adjacent anatomy, e.g., the anterior chamber, the interior of the Schlemm's canal.

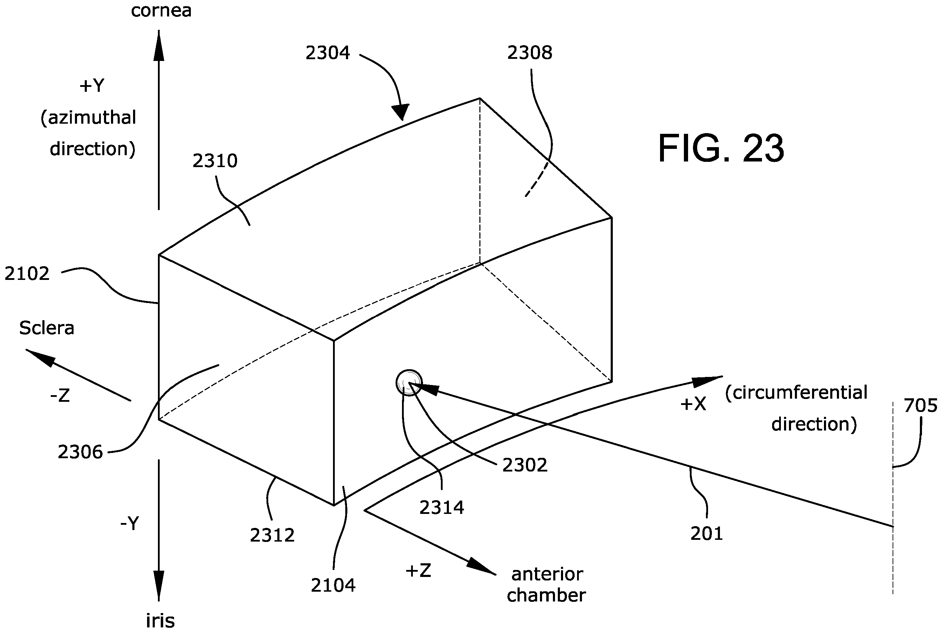

At block **2202** of FIG. 22, and with additional reference to FIG. 23, a focus **2302** of a laser **201** is moved through a target volume **2304** of ocular tissue based on the parameters of a clearing pattern. This moving may include transverse scanning the focus **2302** in the x direction and/or y direction through an XY pattern and axial scanning the focus in the z direction, either in one direction or back and forth. A focus **2302** may be considered to have moved through the target volume **2304** of ocular tissue upon one scan through the clearing pattern, where a scan through a clearing pattern includes a complete transverse scan of the focus **2302** through the XY pattern and a complete axial scan of the focus in the z direction.

The transverse scanning and the axial scanning may occur simultaneously. To these ends, at block **2204,** the focus **2302** is transverse scanned through an XY pattern by being scanned between at least one of: a first circumferential boundary **2306** and a second circumferential boundary **2308** of the target volume **2304** of ocular tissue, and a first azimuthal boundary **2310** and a second azimuthal boundary **2312** of the target volume of ocular tissue. For example, transverse scanning may involve scanning the focus **2302** in the circumferential direction or x direction between a first circumferential boundary **2306** and a second circumferential boundary **2308,** while the focus is fixed in the azimuthal direction or y direction between a first azimuthal boundary **2310** and a second azimuthal boundary **2312.** Transverse scanning may also involve scanning the focus **2302** in the y direction between a first azimuthal boundary **2310** and a second azimuthal boundary **2312** while the focus is fixed in the x direction between a first circumferential boundary **2306** and a second circumferential boundary **2308.**

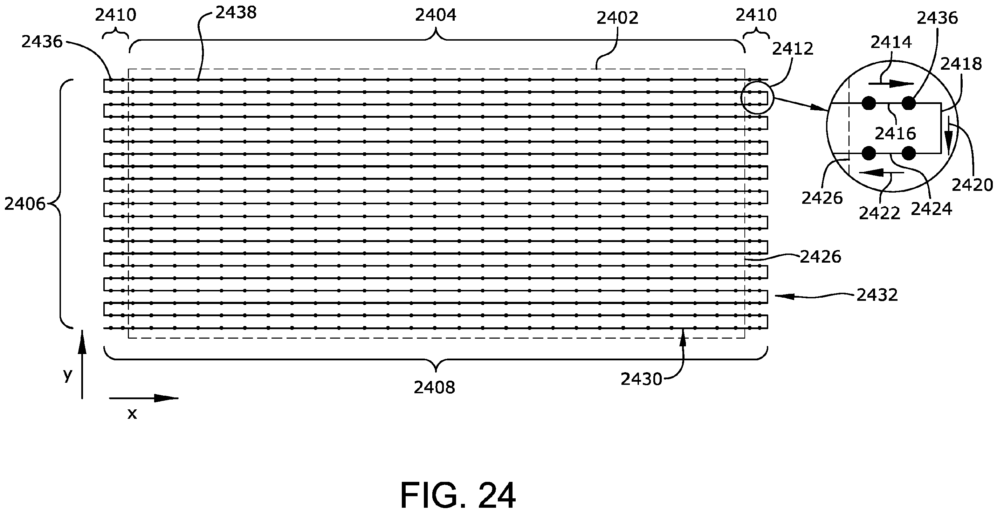

In one approach, the focus **2302** is scanned circumferentially in the x direction along a major scan lines **2430,** and stepped azimuthally in the y direction along a minor scan line **2432** at the end of a circumferential scan line. Typically, treatment pattern dimensions are longer in the circumferential direction than the azimuthal direction. For example, with reference to FIG. 24, a treatment pattern **2402** may be characterized by a major dimension **2404,** e.g., a 500µm circumferential dimension, and a minor dimension **2406,** e.g., a 200µm azimuthal dimension. A full circumferential dimension **2408** comprises the circumferential dimension **2404** and a blanking dimension **2410,** e.g., 50µm, added to each end of the circumferential dimension.

During movement of the focus **2302** through each of a plurality of blanking portions **2412** the laser is blanked, e.g., the output of the laser source is turned off or reduced to a level that does not result in photodisruption of tissue. During a blanking portion **2412** the focus **2302** is scanned along a major scan line **2430** in a first circumferential direction **2414** and decelerates through a deceleration ramp **2416** to the end **2418** of the full scan dimension **2408.** The focus **2302** is then stepped along a minor scan line **2432** in the azimuthal direction **2420,** and then scanned along another major scan line **2430** in a second circumferential direction **2422** opposite the first circumferential direction **2414** and accelerates through an acceleration ramp **2424** to the beginning **2426** of the treatment pattern **2402.**

Scanning the focus **2302** along the longer major axis, e.g., the circumferential x axis, instead of the shorter minor axis, e.g., the azimuthal y axis, is beneficial because such scanning minimizes the total number of deceleration ramps **2416** and acceleration ramps **2424,** thereby minimizing the overall treatment time. Also, blanking on a circumferential scan instead of an azimuthal scan reduces the total number of blanking portions **2412** during a treatment. This provides a more energy efficient treatment during which laser energy is delivered at a series of spots **2434** greater in number and with less frequent blanking, than would be delivered in an azimuthal scan. For example, in FIG. 24, each circumferential scan **2430** includes, going from left to right, two blanked spots **2436,** followed by twenty-seven photodisrupted spots **2438,** followed by two blanked spots **2436,** whereas each azimuthal scan would include only twenty-one photodisrupted spots with two blanking spots on either end.

Returning to FIG. 22, at block **2206,** the focus **2302** of the laser **201** is axial scanned between a distal extent **2102** and a proximal extent **2104** of the target volume **2304** of ocular tissue. The axial scanning includes moving the focus **2302** at an exiting velocity while moving the focus in the direction of the proximal extent **2104,** and moving the focus at an entering velocity while moving the focus in the direction of the distal extent **2102.** In one embodiment, the entering velocity is different from the exiting velocity. For example, the entering velocity may be a multiple of times greater than the exiting velocity. One or both of the entering velocity and the exiting velocity may be constant, thus resulting in a linear type ramping profile, such as the sawtooth profile shown in FIG. 21. Alternatively, at least one of the entering velocity and the exiting velocity may change during the axial scanning, thus resulting in a curved type, e.g., sine wave, ramping profile.

Regarding the transverse scanning and the axial scanning of the focus **2302,** as previously described, while these scans may occur simultaneously, they are asynchronous in that the respective velocities at which the focus is transverse scanned and axial scanned are independent of each other. For example, with reference to FIG. 20, the traverse-scanning velocity has two components, a circumferential scan velocity in the circumferential direction (x direction) and an azimuthal scan velocity in the azimuthal direction (y direction).

The circumferential scan velocity may be given by:
spot separation * laser repetition rate

If the spot separation **2002** is 10 µm and the laser repetition rate is 10 kHz, then the velocity is (10 µm)*(10,000/s) = 100,000 µm/s. This is the velocity the focus **2302** moves in the circumferential direction (x direction).

Continuing with FIG. 20, the azimuthal scan velocity may be given by:
line separation / time to complete one circumferential pass

Accordingly, if the circumferential width or pattern width **2004** of the volume is 750 µm, then the time to complete one circumferential pass is t = (750 µm) / (100,000 µm/s) = 0.0075 s. If the line separation **2006** is 10 µm and the time to complete one circumferential pass is 0.0075 s, the azimuthal velocity is (10 µm) / (0.0075 s) = 1333 µm/s.

In this example, the circumferential velocity is significantly greater than the azimuthal velocity. A quick calculation (based in the spot separation range) shows that the circumferential velocity may range from 20,000 µm/s to 400,000 µm/s.

Note that the exiting velocity **2106** and entering velocity **2108** are fixed independently by the exiting velocity parameter and the entering velocity parameter, and that the range of these velocities as noted above in Table 4 is significantly less than the example circumferential scan velocity, but may be close to the azimuthal velocity.

At block **2208,** the target volume **2304** of ocular tissue is photodisrupted at a plurality of spots **2314** as the focus **2302** is moved through the target volume of ocular tissue. To this end, optical energy is applied to the tissue while the focus **2302** is at the plurality of spots **2314.**

At block **2210,** the process may return to blocks **2202** and **2208,** where the moving and photodisrupting are repeated one or more times as needed. For example, the laser focus may be scanned through a clearing pattern one or more additional times to ensure the focus **2302** has meandered around within the boundaries of the target volume **2304** of ocular tissue enough to fill the volume with spots of photodisruption to form an aperture.

It is possible that the bubbles and debris created by a single progression or cycle of the focus **2302,** also referred to herein as the surgical cut, through the clearing pattern requires repetition through the clearing pattern in order to create a clear aperture. In this case, since the clearing of the bubbles and debris, and progression of the focus **2302** from the boundaries, e.g., the first circumferential boundary **2306** and the second circumferential boundary **2308** of the target volume **2304** of ocular tissue, to the inside of the volume depends on the number of repetitions of the clearing pattern, the number of repetitions through the clearing pattern can be used to control how complete the removal of tissue is from the target volume **2304.** In other words, the number of repetitions control the cutting depth, e.g., the depth of the aperture resulting from the combination of the advancement of the surgical cut and the clearing pattern. A higher number of repetitions of the focus **2302** through the clearing pattern results in deeper penetration into the tissue. The relationship between the number of repetitions through a clearing pattern and the clarity of the aperture, or depth of penetration of the aperture, can be determined empirically in ex vivo tissue studies or in one set of patients, and the relationship can be used to predict the number of repetitions needed in subsequent laser treatments in later patients.

Returning to block **2208,** if the laser focus **2302** has moved through the target volume **2304** of ocular tissue enough times to form an aperture, the process proceeds to block **2212,** where the treatment procedure is ended.

With reference to FIGS. 7-10b, a system **1000** for implementing the method of FIG. 22 includes a first optical subsystem **1001** including a focusing objective **700** configured to be coupled to the eye **1,** and a second optical subsystem **1002** including a laser source **200** configured to output a laser beam **201/701.** The second optical subsystem **1002** also includes a plurality of components **1003** configured to one or more of focus, scan, and direct the laser beam through the focusing objective, toward a target volume **2304** ocular tissue.

The surgical system **1000** also includes a control system **100** coupled to the second optical subsystem **1002.** The control system **100** is configured to control the laser source **200** and plurality of components **1003** to: move a focus **2302** of a laser beam **201** through the target volume **2304** of ocular tissue, and to photodisrupt the target volume of ocular tissue at a plurality of spots **2314** as the focus is moved through the target volume of ocular. The control system **100** moves the focus **2302** of the laser beam **201** by transverse scanning the focus between at least one of: a first circumferential boundary **2306** and a second circumferential boundary **2308** of the target volume **2304** of ocular tissue, and a first azimuthal boundary **2310** and a second azimuthal boundary **2312** of the target volume of ocular tissue, and axial scanning the focus between a distal extent **2102** and a proximal extent **2104** of the target volume of ocular tissue.

A treatment with a surgical laser by the thus disclosed method consists in scanning the focus of the laser in three-dimensional volume within the tissue. The volume is created by scanning the focus a prescribed amount in x, y and x directions. In this manner the tissue is disrupted in a series of layers with area xy and thickness z. However, as a layer or as the entire volume is completed, bubbles and debris may fill the layer or volume. The bubbles or tissue debris may be cleared from the volume by scanning the xy area of a layer while simultaneously oscillating the z depth of the layer. As the laser focus quickly traverses in the z direction, jets of bubbles are ejected from the focal volume with force sufficient to clear any gas bubble or debris from within the volume.

The various aspects of this disclosure are provided to enable one of ordinary skill in the art to practice the present invention. Various modifications to exemplary embodiments presented throughout this disclosure will be readily apparent to those skilled in the art. Thus, the claims are not intended to be limited to the various aspects of this disclosure but are to be accorded the full scope consistent with the language of the claims.

It is to be understood that the embodiments of the invention herein described are merely illustrative of the application of the principles of the invention. Reference herein to details of the illustrated embodiments is not intended to limit the scope of the claims, which themselves recite those features regarded as essential to the invention.

## Claims

1. A system for treating a target volume of ocular tissue of an irido-corneal angle of an eye, the system comprising:
a first optical subsystem including a focusing objective configured to be coupled to the eye;
a second optical subsystem including a laser source configured to output a laser beam, and a plurality of components configured to one or more of focus, scan, and direct the laser beam through the focusing objective, toward the target volume of ocular tissue; and
a control system coupled to the second optical subsystem and configured to control the second optical subsystem to:
move a focus of a laser through the target volume of ocular tissue, by being further configured to simultaneously:
transverse scan the focus between at least one of: a first circumferential boundary and a second circumferential boundary of the target volume of ocular tissue, and a first azimuthal boundary and a second azimuthal boundary of the target volume of ocular tissue, and
axial scan the focus between a distal extent and a proximal extent of the target volume of ocular tissue; and
photodisrupt the target volume of ocular tissue at a plurality of spots as the focus is moved through the target volume of ocular tissue.

2. The system of claim 1, wherein the control system is further configured to control the second optical subsystem to move the focus through the target volume of ocular tissue and photodisrupt the target volume of ocular tissue a plurality times.

3. The system of claim 1, wherein the control system is further configured to control the second optical subsystem to transverse scan the focus between both of the first circumferential boundary and the second circumferential boundary, and the first azimuthal boundary and the second azimuthal boundary.

4. The system of claim 3, wherein:
a major dimension of a major scan line between one of the first circumferential boundary and the second circumferential boundary, and the first azimuthal boundary and the second azimuthal boundary is greater than a minor dimension of a minor scan line between the other of the first circumferential boundary and the second circumferential boundary, and the first azimuthal boundary and the second azimuthal boundary, and
the focus is scanned along the major scan line to an end of the major scan line before being moved along the minor scan line.

5. The system of claim **1,** wherein the control system is configured to control the second optical subsystem to axial scan the focus by:
moving the focus at an exiting velocity while moving the focus in the direction of the proximal extent, and
moving the focus at an entering velocity while moving the focus in the direction of the distal extent.

6. The system of claim 5, wherein the entering velocity is different from the exiting velocity.

7. The system of claim 5, wherein one or both of the entering velocity and the exiting velocity are constant.

8. The system of claim 5, wherein at least one of the entering velocity and the exiting velocity changes during the axial scanning.

9. The system of claim 1, wherein the target volume of ocular tissue is entirely within ocular tissue.

10. The system of claim 1, wherein at least a portion of the target volume of ocular tissue encompasses adjacent anatomy.

11. The system of claim 1, wherein the control system is configured to control the second optical subsystem to photodisrupt tissue by being further configured to control the laser to apply optical energy to the tissue while the focus is the plurality of spots.

12. The system of claim 1, wherein the second optical subsystem includes an imaging apparatus and the control system is further configured to control the second optical subsystem to:
detect the distal extent of the target volume of ocular tissue; and
detect the proximal extent of the target volume of ocular tissue.

13. The system of claim 12, wherein the distal extent of the target volume of ocular tissue and the proximal extent of the target volume of ocular tissue are detected based on one or more images of ocular tissue.

14. The system of claim 12, wherein the imaging apparatus comprises one of:
an optical imaging apparatus configured to obtain the one or more images of ocular tissue;
a multiphoton imaging apparatus configured to obtain the one or more images of ocular tissue; and
an opto-mechanical imaging apparatus configured to obtain the one or more images of ocular tissue.

## Patentansprüche

1. System zum Behandeln eines Zielvolumens von Augengewebe eines Irido-Hornhaut-Winkels eines Auges, wobei das System Folgendes umfasst:
ein erstes optisches Teilsystem, das ein Fokussierobjektiv beinhaltet, das konfiguriert ist, um mit dem Auge gekoppelt zu werden;
ein zweites optisches Teilsystem, das eine Laserquelle beinhaltet, die konfiguriert ist, um einen Laserstrahl auszugeben, und eine Vielzahl von Komponenten, die konfiguriert sind, um eines oder mehrere von zu fokussieren, abzutasten und den Laserstrahl durch das Fokussierobjektiv auf das Zielvolumen von Augengewebe zu richten; und
ein Steuersystem, das mit dem zweiten optischen Teilsystem gekoppelt und konfiguriert ist, um das zweite optische Teilsystem zu steuern, um:
einen Fokus eines Lasers durch das Zielvolumen von Augengewebe zu bewegen, indem es ferner konfiguriert ist, um gleichzeitig:
den Fokus zwischen mindestens einem von: einer ersten Umfangsgrenze und einer zweiten Umfangsgrenze des Zielvolumens von Augengewebe und einer ersten azimutalen Grenze und einer zweiten azimutalen Grenze des Zielvolumens von Augengewebe transversal abzutasten, und
den Fokus zwischen einer distalen Ausdehnung und einer proximalen Ausdehnung des Zielvolumens von Augengewebe axial abzutasten; und
das Zielvolumen von Augengewebe an einer Vielzahl von Punkten zu photounterbrechen, während der Fokus durch das Zielvolumen von Augengewebe bewegt wird.

2. System nach Anspruch 1, wobei das Steuersystem ferner konfiguriert ist, um das zweite optische Teilsystem zu steuern, um den Fokus durch das Zielvolumen von Augengewebe zu bewegen und das Zielvolumen von Augengewebe eine Vielzahl von Malen zu photounterbrechen.

3. System nach Anspruch 1, wobei das Steuersystem ferner konfiguriert ist, um das zweite optische Teilsystem zu steuern, um den Fokus zwischen sowohl der ersten Umfangsgrenze als auch der zweiten Umfangsgrenze und der ersten azimutalen Grenze und der zweiten azimutalen Grenze transversal abzutasten.

4. System nach Anspruch 3, wobei:
eine Hauptabmessung einer Hauptabtastlinie zwischen einer von der ersten Umfangsgrenze und der zweiten Umfangsgrenze und der ersten azimutalen Grenze und der zweiten azimutalen Grenze größer ist als eine Nebenabmessung einer Nebenabtastlinie zwischen der anderen von der ersten Umfangsgrenze und der zweiten Umfangsgrenze und der ersten azimutalen Grenze und der zweiten azimutalen Grenze, und
der Fokus entlang der Hauptabtastlinie bis zu einem Ende der Hauptabtastlinie abgetastet wird, bevor er entlang der Nebenabtastlinie bewegt wird.

5. System nach Anspruch 1, wobei das Steuersystem konfiguriert ist, um das zweite optische Teilsystem zu steuern, um den Fokus axial abzutasten durch:
Bewegen des Fokus mit einer Austrittsgeschwindigkeit, während der Fokus in die Richtung der proximalen Ausdehnung bewegt wird, und
Bewegen des Fokus mit einer Eintrittsgeschwindigkeit, während der Fokus in die Richtung der distalen Ausdehnung bewegt wird.

6. System nach Anspruch 5, wobei sich die Eintrittsgeschwindigkeit von der Austrittsgeschwindigkeit unterscheidet.

7. System nach Anspruch 5, wobei eine oder beide der Eintrittsgeschwindigkeit und der Austrittsgeschwindigkeit konstant sind.

8. System nach Anspruch 5, wobei sich mindestens eine der Eintrittsgeschwindigkeit und der Austrittsgeschwindigkeit während des axialen Abtastens ändert.

9. System nach Anspruch 1, wobei sich das Zielvolumen von Augengewebe vollständig innerhalb von Augengewebe befindet.

10. System nach Anspruch 1, wobei mindestens ein Abschnitt des Zielvolumens von Augengewebe angrenzende Anatomie umschließt.

11. System nach Anspruch 1, wobei das Steuersystem konfiguriert ist, um das zweite optische Teilsystem zu steuern, um Gewebe zu photounterbrechen, indem es ferner konfiguriert ist, um den Laser zu steuern, um optische Energie auf das Gewebe anzuwenden, während der Fokus die Vielzahl von Punkten ist.

12. System nach Anspruch 1, wobei das zweite optische Teilsystem eine Bildgebungsvorrichtung beinhaltet und das Steuersystem ferner konfiguriert ist, um das zweite optische Teilsystem zu steuern, um:
die distale Ausdehnung des Zielvolumens von Augengewebe zu erfassen; und
die proximale Ausdehnung des Zielvolumens von Augengewebe zu erfassen.

13. System nach Anspruch 12, wobei die distale Ausdehnung des Zielvolumens von Augengewebe und die proximale Ausdehnung des Zielvolumens von Augengewebe basierend auf einem oder mehreren Bildern von Augengewebe erfasst werden.

14. System nach Anspruch 12, wobei die Bildgebungsvorrichtung eines der Folgenden umfasst:
eine optische Bildgebungsvorrichtung, die konfiguriert ist, um das eine oder die mehreren Bilder von Augengewebe zu erhalten;
eine Multiphotonen-Bildgebungsvorrichtung, die konfiguriert ist, um das eine oder die mehreren Bilder von Augengewebe zu erhalten; und
eine optomechanische Bildgebungsvorrichtung, die konfiguriert ist, um das eine oder die mehreren Bilder von Augengewebe zu erhalten.

## Revendications

1. Système pour traiter un volume cible de tissu oculaire d'un angle irido-cornéen d'un oeil, le système comprenant :
un premier sous-système optique comprenant un objectif de focalisation configuré pour être couplé à l'oeil ;
un second sous-système optique comprenant une source laser configurée pour émettre un faisceau laser, et une pluralité de composants configurés pour un ou plusieurs parmi la focalisation, le balayage, et la direction du faisceau laser à travers l'objectif de focalisation, vers le volume cible de tissu oculaire ; et
un système de commande couplé au second sous-système optique et configuré pour commander le second sous-système optique pour :
déplacer une focalisation d'un laser à travers le volume cible de tissu oculaire, en étant en outre configuré pour simultanément :
balayer transversalement la focalisation entre au moins l'une parmi : une première limite circonférentielle et une seconde limite circonférentielle du volume cible de tissu oculaire, et une première limite azimutale et une seconde limite azimutale du volume cible de tissu oculaire, et
balayer axialement la focalisation entre une étendue distale et une étendue proximale du volume cible de tissu oculaire ; et
photoperturber le volume cible de tissu oculaire au niveau d'une pluralité de spots lorsque la focalisation est déplacée à travers le volume cible de tissu oculaire.

2. Système selon la revendication 1, dans lequel le système de commande est en outre configuré pour commander le second sous-système optique pour déplacer la focalisation à travers le volume cible de tissu oculaire et photoperturber le volume cible de tissu oculaire une pluralité de fois.

3. Système selon la revendication 1, dans lequel le système de commande est en outre configuré pour commander le second sous-système optique pour balayer transversalement la focalisation entre à la fois la première limite circonférentielle et la seconde limite circonférentielle, et la première limite azimutale et la seconde limite azimutale.

4. Système selon la revendication 3, dans lequel :
une dimension majeure d'une ligne de balayage majeure entre l'une parmi la première limite circonférentielle et la seconde limite circonférentielle, et la première limite azimutale et la seconde limite azimutale est supérieure à une dimension mineure d'une ligne de balayage mineure entre l'autre parmi la première limite circonférentielle et la seconde limite circonférentielle, et la première limite azimutale et la seconde limite azimutale, et
la focalisation est balayée le long de la ligne de balayage majeure jusqu'à une extrémité de la ligne de balayage majeure avant d'être déplacée le long de la ligne de balayage mineure.

5. Système selon la revendication 1, dans lequel le système de commande est configuré pour commander le second sous-système optique pour balayer axialement la focalisation en :
déplaçant la focalisation à une vitesse de sortie tout en déplaçant la focalisation dans la direction de l'étendue proximale, et
déplaçant la focalisation à une vitesse d'entrée tout en déplaçant la focalisation dans la direction de l'étendue distale.

6. Système selon la revendication 5, dans lequel la vitesse d'entrée est différente de la vitesse de sortie.

7. Système selon la revendication 5, dans lequel l'une ou les deux de la vitesse d'entrée et de la vitesse de sortie sont constantes.

8. Système selon la revendication 5, dans lequel au moins l'une de la vitesse d'entrée et de la vitesse de sortie change pendant le balayage axial.

9. Système selon la revendication 1, dans lequel le volume cible de tissu oculaire est entièrement à l'intérieur du tissu oculaire.

10. Système selon la revendication 1, dans lequel au moins une partie du volume cible de tissu oculaire englobe une anatomie adjacente.

11. Système selon la revendication 1, dans lequel le système de commande est configuré pour commander le second sous-système optique pour photoperturber le tissu en étant en outre configuré pour commander le laser pour appliquer une énergie optique au tissu tandis que la focalisation est la pluralité de spots.

12. Système selon la revendication 1, dans lequel le second sous-système optique comprend un appareil d'imagerie et le système de commande est en outre configuré pour commander le second sous-système optique pour :
détecter l'étendue distale du volume cible de tissu oculaire ; et
détecter l'étendue proximale du volume cible de tissu oculaire.

13. Système selon la revendication 12, dans lequel l'étendue distale du volume cible de tissu oculaire et l'étendue proximale du volume cible de tissu oculaire sont détectées sur la base d'une ou plusieurs images de tissu oculaire.

14. Système selon la revendication 12, dans lequel l'appareil d'imagerie comprend l'un parmi :
un appareil d'imagerie optique configuré pour obtenir les une ou plusieurs images de tissu oculaire ;
un appareil d'imagerie multiphotonique configuré pour obtenir les une ou plusieurs images de tissu oculaire ; et
un appareil d'imagerie optomécanique configuré pour obtenir les une ou plusieurs images de tissu oculaire.
